# EUROPEAN PATENT SPECIFICATION

(11) **EP 1 599 148 B1**
(45) Date of publication and mention of the grant of the patent: **20.04.2011**
(21) Application number: 04714573.5
(22) Date of filing: 25.02.2004
(51) Int. Cl.: A61B 19/00, A61B 17/86, A61B 17/68

(54) **FIDUCIAL MARKER DEVICES**
BEZUGSMARKIERUNGSVORRICHTUNGEN
SYSTEMES DE REPERES FIDUCIELS

(30) Priority: 25.02.2003 US 374677; 04.06.2003 US 454786; 17.10.2003 US 688801
(43) Date of publication of application: 30.11.2005
(73) Proprietor: Medtronic Image-Guided Neurologics, Inc., Minneapolis, MN 55432 (US)
(72) Inventor: SOLAR, Matthew, S., Indialantic, FL 32903 (US); BRIDGES, Thomas, L., Melbourn e Beach, FL 32951 (US); LEE, David, Melbourne Beach, FL 32951 (US); FREAS, Mark, Palm Bay, FL 32909 (US); MAZZOCCHI, Rudy, A., Indian Harbor Beach, FL 32937 (US)
(74) Representative: Collins, John David
(86) International application number: PCT/US2004/005470
(87) International publication number: WO 2004/075768

(56) References cited:
- EP-A- 0 787 467
- EP-A- 0 813 846
- EP-A- 0 820 736
- EP-A- 1 033 113
- EP-A- 1 033 113
- EP-A- 1 249 207
- EP-A- 1 249 207
- WO-A-01/78015
- WO-A-97/09929
- WO-A-98/38908
- WO-A-99/11998
- WO-A-99/16352
- WO-A1-99/40869
- DE-U1- 9 112 356
- RU-C1- 2 026 648
- US-A- 4 630 375
- US-A- 4 943 293
- US-A- 5 368 030
- US-A- 5 368 030
- US-A- 5 394 457
- US-A- 5 397 329
- US-A- 5 397 329
- US-A- 5 469 847
- US-A- 5 636 255
- US-A- 5 636 255
- US-A1- 5 683 217
- US-A1- 6 000 892
- US-A1- 2001 010 004
- US-A1- 2001 010 004
- US-A1- 2001 027 271
- US-A1- 2002 052 610
- US-B1- 6 351 659
- US-B1- 6 351 659
- US-B1- 6 402 757
- US-B1- 6 430 434
- US-B1- 6 430 434
- US-B1- 6 499 488
- US-B1- 6 499 488

## Description

### FIELD OF THE INVENTION

This document relates generally to imaging and/or locating a subject, such as for performing surgical intervention, and more specifically, but not by way of limitation, to fiducial marker devices and associated tools and methods.

### BACKGROUND

Fiducial markers that can be located and recognized by an imaging system or other system are useful in neurosurgery and other applications. Examples of imaging system modalities include, among other things, magnetic resonance imaging (MRI), computed tomography (CT), positron emission tomography (PET), and single photon emission computed tomography (SPECT).

For example, in one technique, multiple fiducial markers are screwed into the patient's skull to define landmarks recognizable by an imaging system. The imaging system is used to obtain one or more preoperative images of the patient's brain. Recognizable images of the fiducial markers appear on such preoperative images. Such a bone-anchored fiducial marker typically includes an externally threaded bone-screw portion, which is driven into the skull. A threaded shaft rises up and out of the skull from the bone-screw. The threaded shaft typically receives a screwed-on imagable sphere that is visible on an MRI or CT image. The multiple fiducial markers on the patient's skull define landmarks on preoperative images that are useful to the physician for planning entry coordinates on the patient's skull and for planning a trajectory to a target location in the brain. An image-guided surgical workstation uses these preoperative images and the planning data to guide the neurosurgeon while actually performing the subsequent surgical procedure.

After the preoperative planning phase, the patient is brought into the operating room so that the planned surgical procedure can be performed. On the operating table, the patient's skull is clamped in a head-frame or otherwise immobilized. In order to use the preoperative images provided by the image-guided workstation to guide the surgeon during the surgical procedure, the patient's skull must first be "registered" to the preoperative images. The registration creates an association between (1) the actual physical location of the fiducial markers on the patient's skull in the operating room and (2) the locations of the images of the fiducial markers visible on the preoperatively-obtained images. This allows mapping between the actual space in which the patient is located to the space defined by the preoperative images.

According to one registration technique, a "wand" is used to perform this patient registration. The wand typically includes multiple light-emitting diode (LED) locators or reflective locators, which are visible to an infrared camera or other detector of an optical positioning system in the operating room. The camera and optical positioning system are operatively connected to the image-guided workstation. The locators define the position of the wand in the operating room, including the position of a sharp tip portion of the wand, which is in a known physical relationship to the locators. To register the patient, the imagable, spheres are unscrewed from the fiducial marker shafts, and replaced by respective "divots" that are sized and shaped to receive the wand tip. These divots are screwed or otherwise engaged onto the respective fiducial marker shafts, such that when the wand tip is received into the maximum depression point of the divot, the wand tip then corresponds to the same location as the center of the imagable sphere when the imagable sphere was screwed onto the fiducial marker shaft. A reference divot is typically also present in the operating room at a known location, such as attached to the operating table or the patient's skull-immobilizing head-frame. During the patient registration process, the surgeon touches the wand tip to the reference divot (to provide an absolute positional reference to the image-guided workstation), and then to each fiducial marker divot. This permits the image-guided workstation to correlate the actual physical location of the patient's skull to the preoperative images. The physician can then use the wand, in conjunction with the preoperative images provided by the image-guided workstation, to locate an appropriate entry point and trajectory to the target in the brain. Such devices are known from US-A-2001/0027271.

The present inventors have recognized that problems with the above registration procedure include patient discomfort caused by the presence of the fiducial markers, increased trauma to the patient resulting from using multiple fiducial markers screwed into different locations of the patient's skull, the difficulty of unscrewing the imaging spheres and replacing them with the registration divots, a limited field of view of the camera used in the operating room, and the difficulty of constructing a multi-modal fiducial marker that can be recognized by more than one imaging modality or positioning system.
Moreover, the present inventors have recognized the desirability of streamlining the registration process to reduce its time and cost. For these and other reasons, which will become apparent upon reading the following detailed description and viewing the drawings that form a part thereof, the present inventors have recognized an unmet need for improved fiducial marker devices, tools, and methods.

### SUMMARY

One aspect of the present invention provides an apparatus comprising: an imageable fiducial marker including an imageable sphere having a receptacle; a base, the base comprising proximal and distal ends, the distal end including external threads, the proximal end attached to said imageable fiducial marker, and an imageable fiducial marker portion; wherein the receptacle of the imageable fiducial marker is sized and shaped to receive the imageable fiducial marker portion and also is sized and shaped to receive a positioning instrument.

A further aspect of the invention provides a system comprising the aforementioned apparatus; a light source configured to illuminate positioning locators of the positioning instrument; and a detector configured to detect light reflected from the positioning locators.

A further aspect of the present invention provides a method comprising_{'} attaching imageable spheres to respective ones of a plurality of bases that have been screwed into at least one surface of a subject; imaging a volume including the imageable spheres; removing respective portions of the imageable spheres to permit access to centers of the imageable spheres; and registering a subject to the images, including touching the centers of the imageable spheres with a positioning instrument.

One example illustrates a combined computed tomography (CT) imagable fiducial locator head, an integral bone screw, and an integral divot for receiving a positioning wand of an image-guided surgical (IGS) workstation. A further example includes a fluid/gel-absorbing coating or cover into which a magnetic resonance (MR) imagable fluid is introduced, thereby permitting both CT and MR imaging. Protective caps and collars may be used to protect the fiducial marker from mechanical impact and/or to guide the fiducial marker during affixation- A bull's-eye or other template is used to select a center of a substantially spherical fiducial marker head on an image, such as for use during patient registration. Another example includes a positioning instrument with a cap that mates directly to an imageable sphere to perform registration. Another example includes a jointed positioning instrument that, when placed In a base, pivots about a location defined by a center of the imageable sphere when it was in the base Another example includes a fiducial marker with two imageable spheres defining a line intersecting a desired point on the base. Another example includes a base with a receptacle for receiving a positioning instrument. Another example includes an imageable sphere with a removable imageable portion to allow access to a center of the imageable sphere by a positioning instrument.

### BRIEF DESCRIPTION OF THE DRAWINGS

In the drawings, which are not necessarily drawn to scale, like numerals describe substantially similar components throughout the several views. Like numerals having different letter suffixes represent different instances of substantially similar components. The drawings illustrate generally, by way of example, but not by way of limitation, various devices, methods and systems discussed in the present document.
Figure 1A is a schematic diagram illustrating generally one example of an imagable fiducial marker that includes a built-in conical divot or other male or female receptacle, or the like.
Figure 1B is a schematic diagram illustrating generally one example of an imagable fiducial marker that omits the divot illustrated in Figure 1A, but which is both locatable by a remote positioning system and imagable by one or more imaging modalities.
Figure 2A is a schematic diagram illustrating generally an alternative example of a fiducial marker that includes a cylindrical imaging fiducial locator and a conical or other divot or other receptacle for receiving a positioning wand tip or the like.
Figure 28 is a schematic diagram illustrating generally one example of an imagable fiducial marker that omits the divot illustrated in Figure 2A, but which is both locatable by a remote positioning system and imagable by one or more imaging modalities.

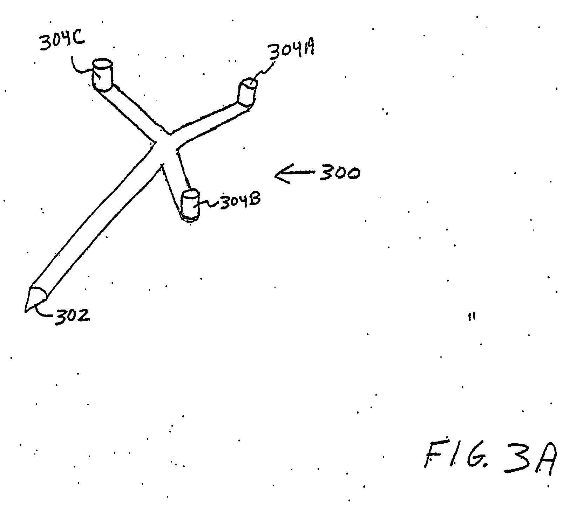

Figure 3A is a schematic diagram illustrating generally one example of a positioning wand for use in conjunction with a remotely-located camera or other like device of an optical positioning system, such as can be coupled to an image- guided surgical workstation in an operating room.

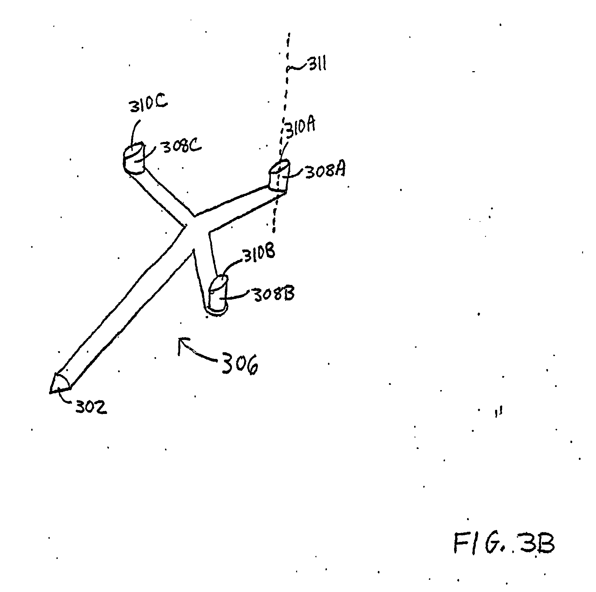

Figure 3B is a schematic diagram, similar in certain respects to Figure 3A, illustrating generally one example of a positioning wand including energy reflective surfaces that are capable of being oriented or aimed toward a remote detector.

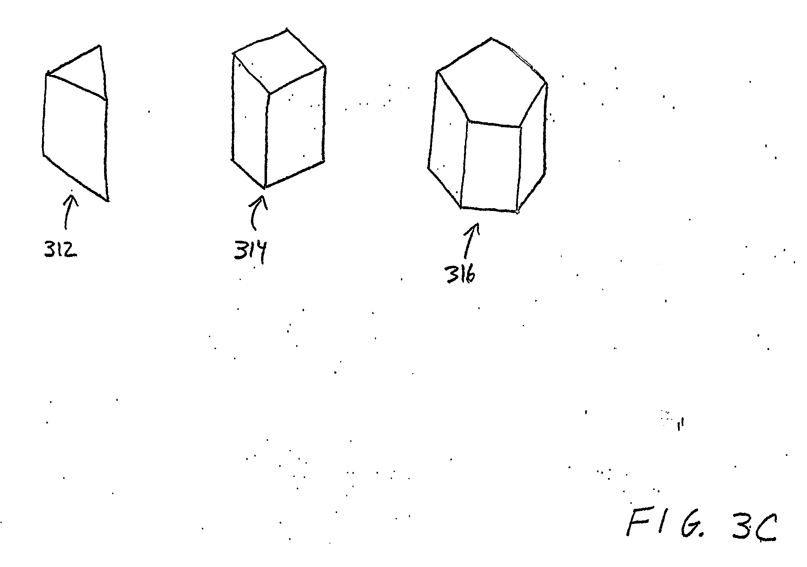

Figure 3C is a perspective view schematic diagram illustrating generally, by way of example, but not by way of limitation, certain generally "cylindrical" columnar structures having faceted lateral peripheral surfaces.

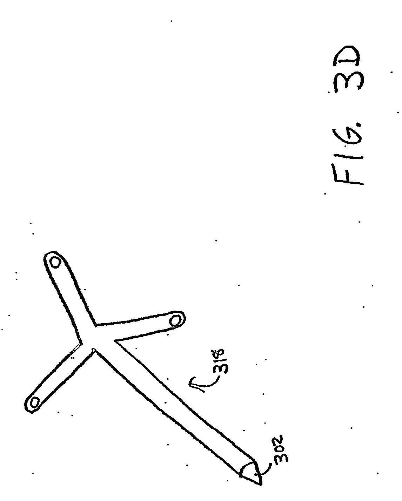

Figure 3D is a schematic diagram illustrating generally an example of a positioning wand with flat disk-shaped pieces of reflective tape are attached in a known configuration.

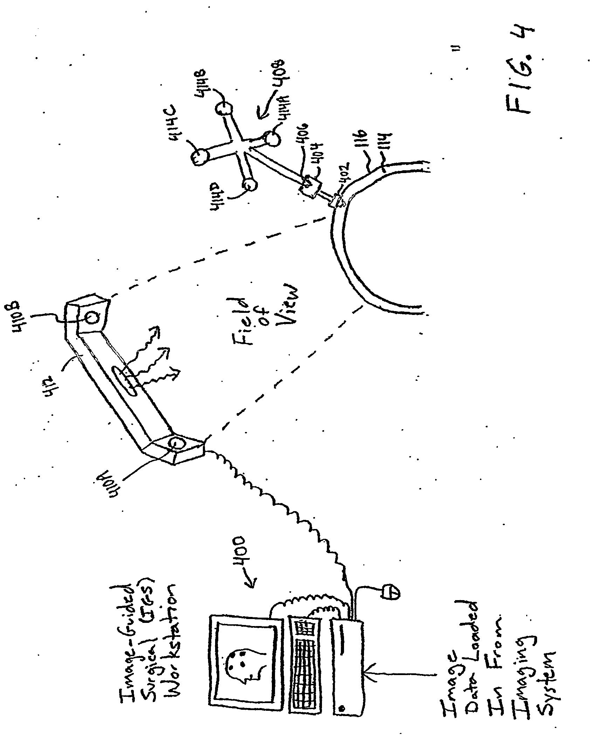

Figure **4** is a schematic diagram illustrating generally, by way of example, but not by way of limitation, an image guided surgical (IGS) computer workstation to which an optical positioning system is coupled.

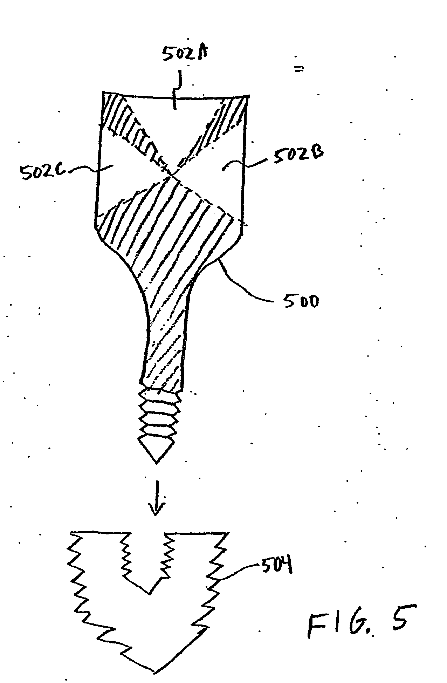

Figures **5** is a schematic diagram illustrating generally a unitary divot assembly that includes multiple divots.

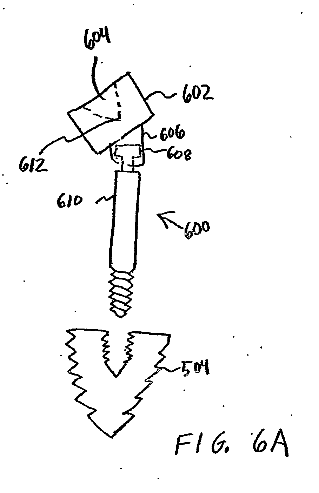

Figure **6A** is a schematic diagram illustrating generally a divot assembly that includes a swiveling tilted head carrying a conical or other divot or the like.

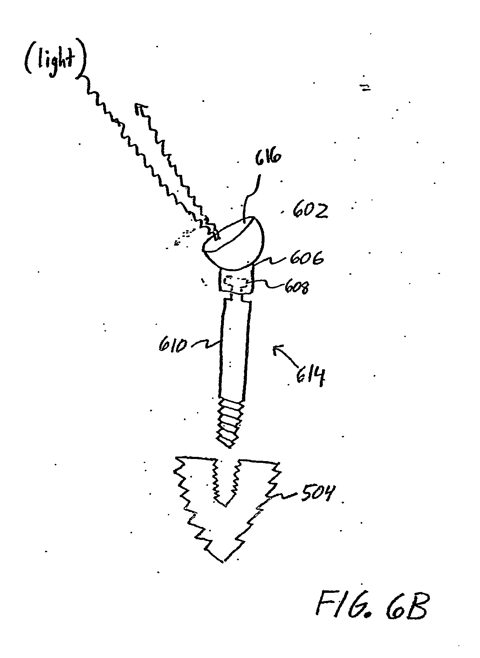

Figure **6B** is a schematic diagram illustrating generally a locator assembly that includes a swiveling tilted head including a surface that reflects electromagnetic energy.

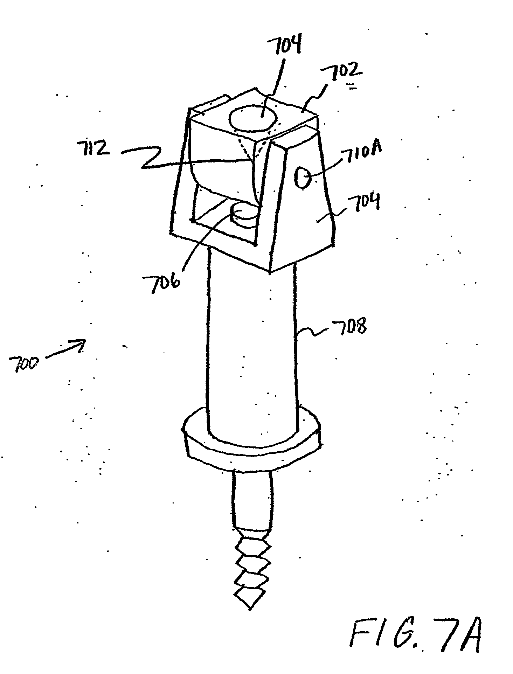

Figure **7A** is a schematic diagram illustrating generally a divot assembly that includes a swiveling and pivotable head carrying a conical or other divot.

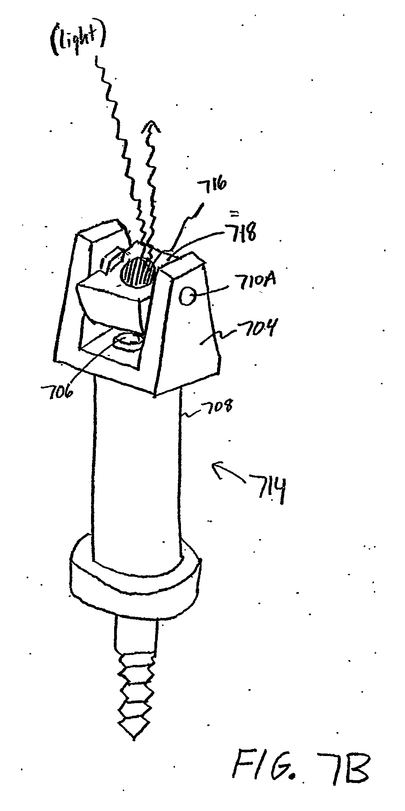

Figure **7B** is a schematic diagram illustrating generally a divot assembly that includes a swiveling and pivotable head including a surface that reflects electromagnetic energy.

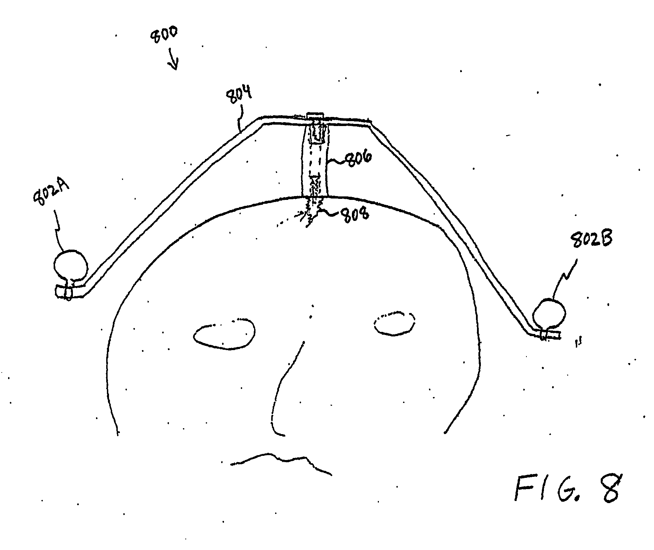

Figure **8** is a schematic diagram illustrating conceptually a fiducial marker carrier that is attachable to (and also detachable from) a single location on the patient's skull, thereby reducing trauma to the patient.

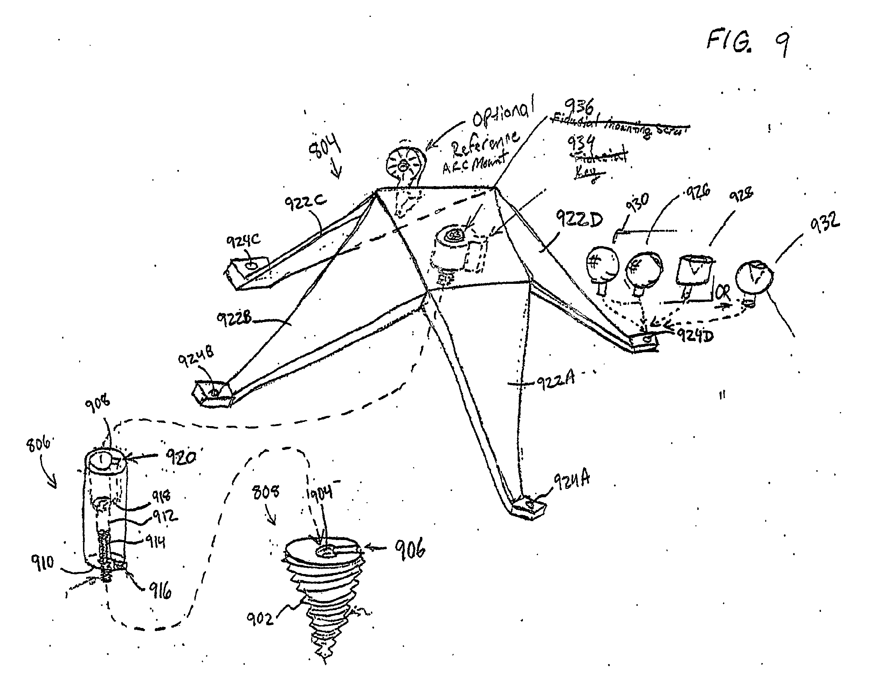

Figure **9** is an exploded view schematic diagram illustrating generally one example of the carrier, including a frame, a post, and a base.

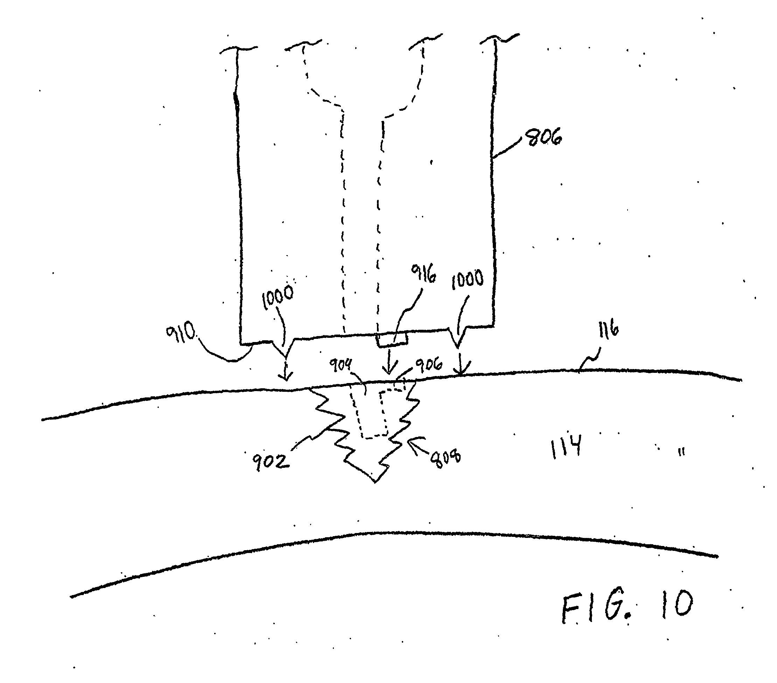

Figure **10** is a schematic diagram illustrating a portion of a fiducial marker carrier that includes at least one antirotational spike for engaging the surface of the skull.

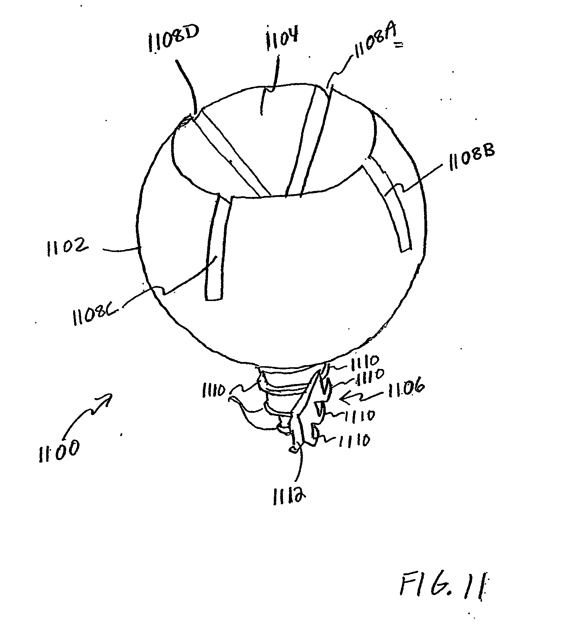

FIG. **11** is a perspective view of an alternative example of a fiducial marker.

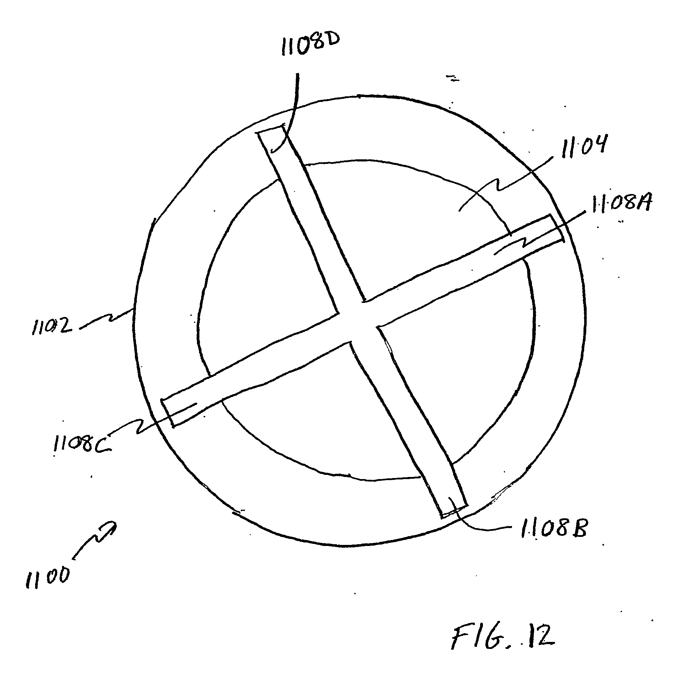

FIG. **12** is a top view of the fiducial marker illustrated in FIG. **11****.**

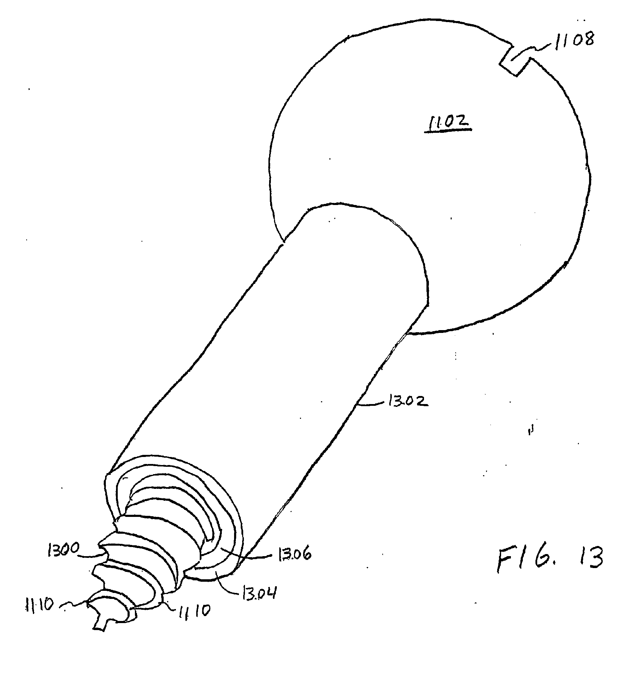

FIG. **13** is a perspective view of modified unitary fiducial marker.

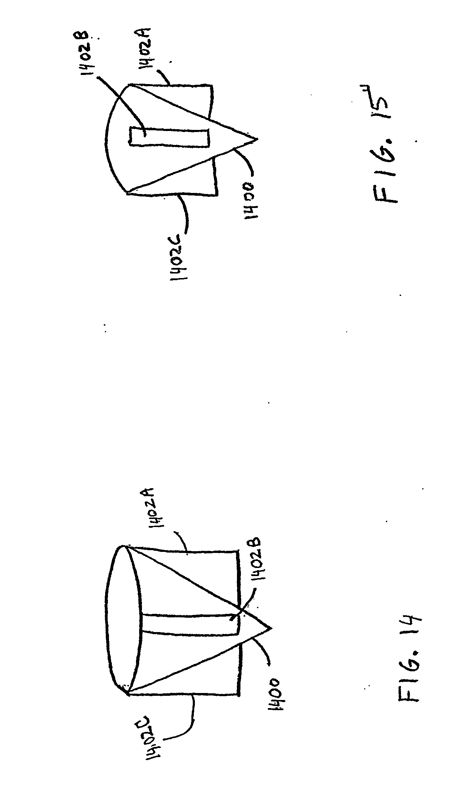

FIG. **14** is a perspective view of an optional imagable plug.
FIG. **15** is a side view of the optional imagable plug of FIG.**14**.

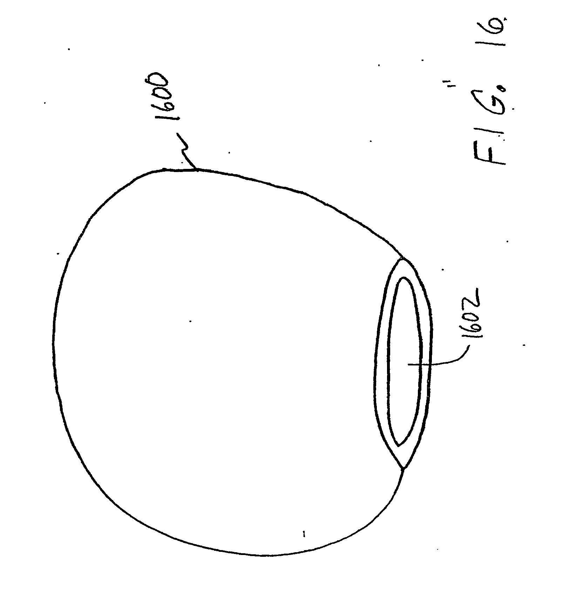

FIG. **16** is a perspective view of an optional fluid absorbing cover (or coating).

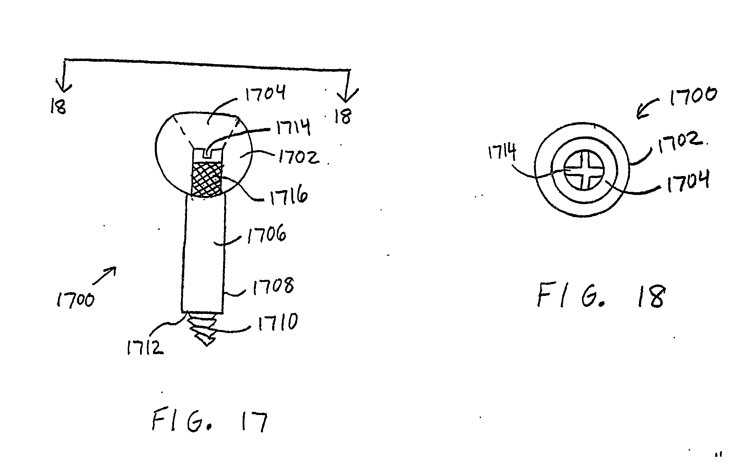

FIG. **17** is a side cross-sectional view of an alternative example of a fiducial marker.
FIG. **18** is a top view of an the fiducial marker of FIG. **17**.

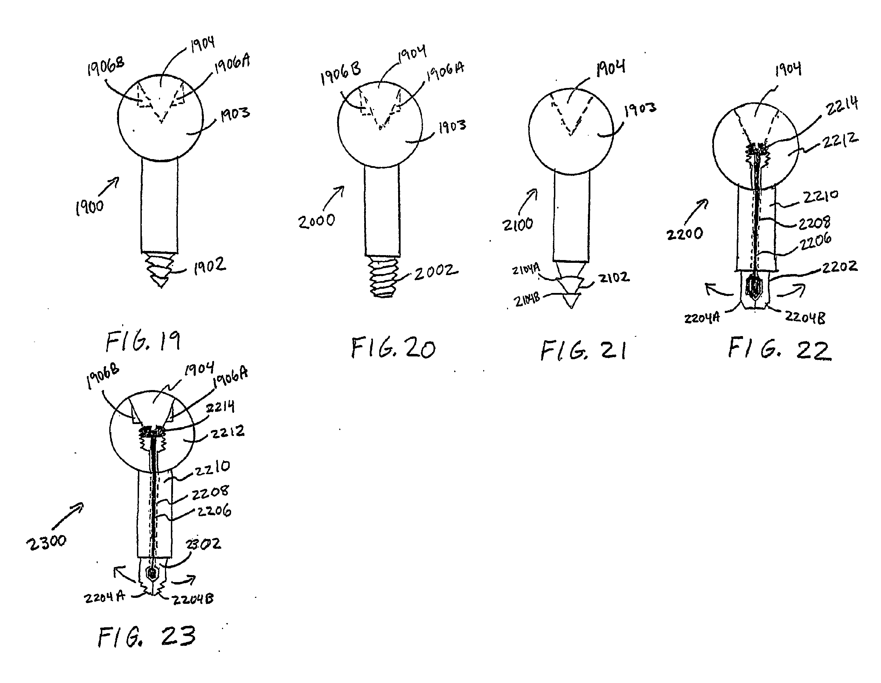

FIG. **19** illustrates a side view of a fiducial marker that includes a self drilling and self-tapping threaded distal tip portion.
FIG. **20** illustrates a side view of a fiducial marker that includes a threaded distal tip portion that need not be self-tapping and/or self-drilling.
FIG. **21** illustrates a side view of a fiducial marker that includes a barbed distal tip portion.
FIG. **22** illustrates a side view of a fiducial marker having a distal tip portion that includes tangs, or another laterally expandable retention element.
FIG. **23** illustrates a side view of a fiducial marker including a laterally expandable retention element and also having a self-tapping and/or self-drilling externally threaded distal tip portion.

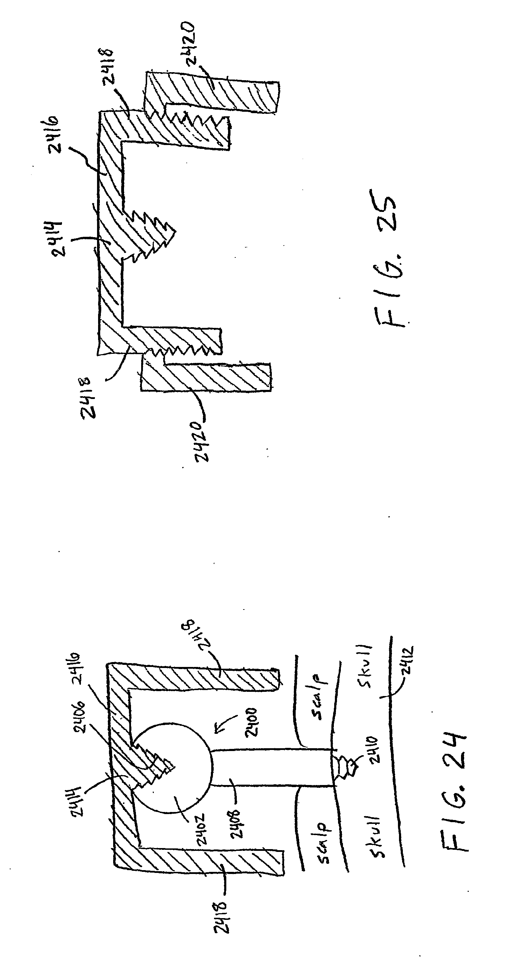

FIG. **24** is a side cross-sectional view of a fiducial marker having a protective cap.
FIG. **25** is a side cross-sectional view of a protective cap with an adjustable-height skirt.

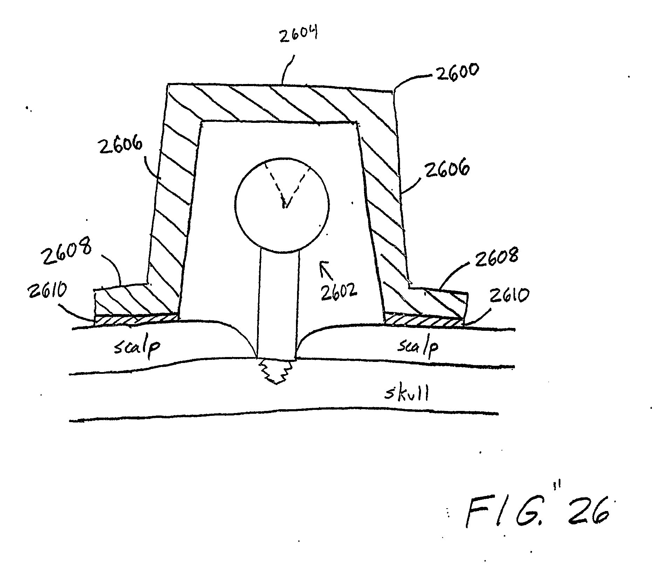

FIG. **26** is a side cross-sectional view of a protective cap disposed about a fiducial marker.

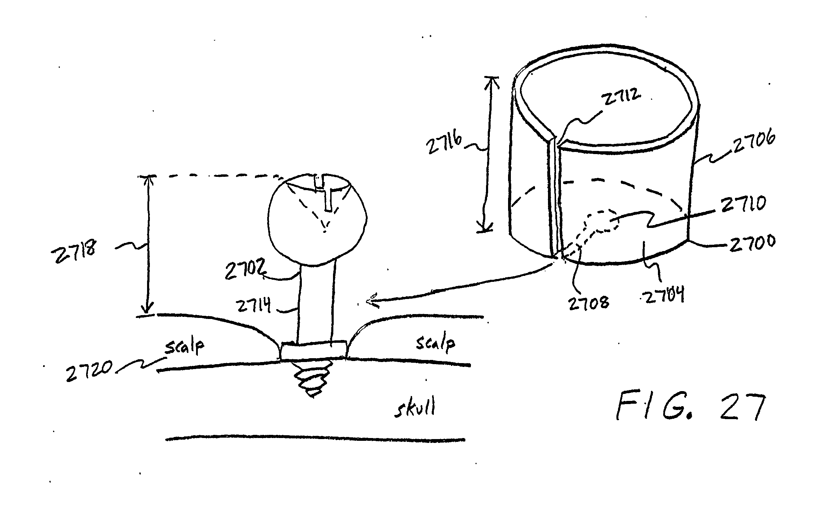

FIG. **27** is a perspective view of a protective collar that can be disposed about a fiducial marker that has been affixed to a subject's skull.

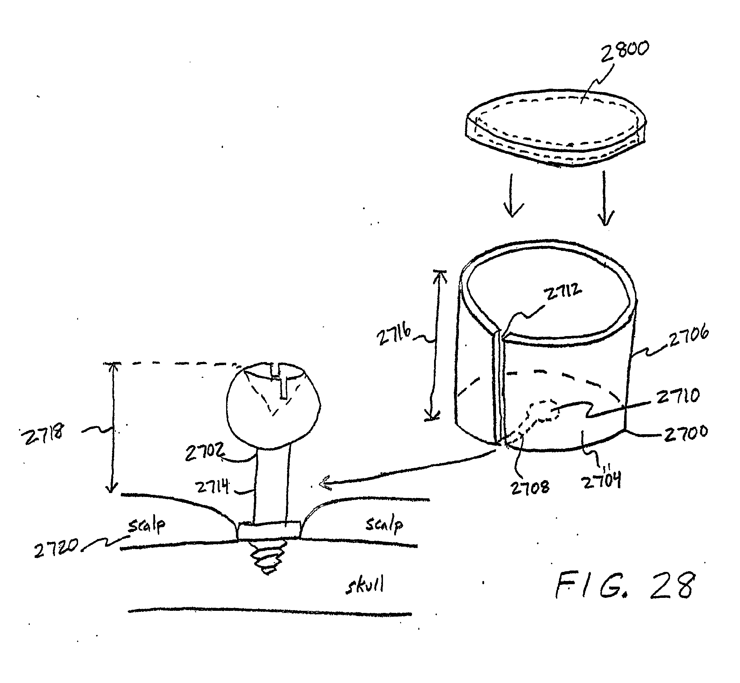

FIG. **28** is a perspective view of a protective collar and cap.

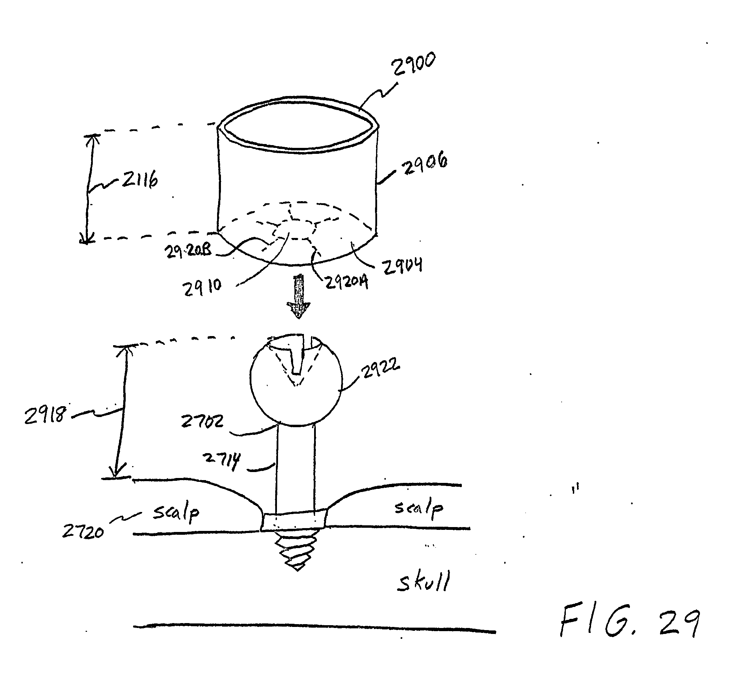

FIG. **29** is a perspective view of an alternate example of a protective collar that can be slipped over a fiducial marker.

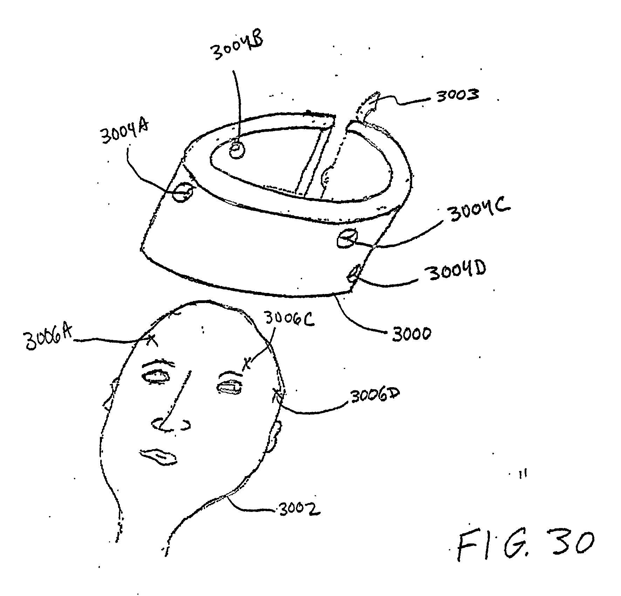

FIG. **30** is a perspective view illustrating an example of a headband for protecting fiducial markers from mechanical impact.

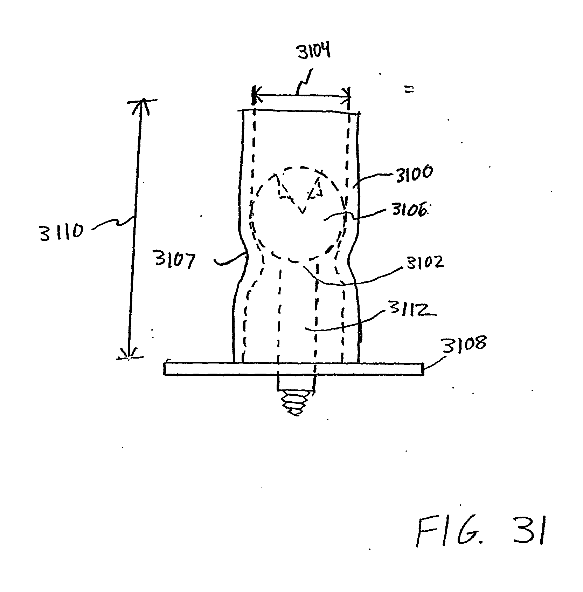

FIG. **31** is a side view illustrating an example of a tubular protective guide collar.

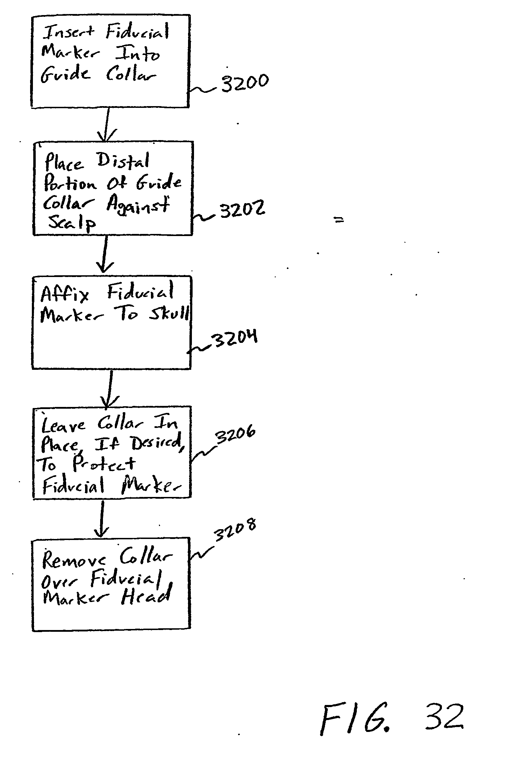

FIG. **32** is a flow chart illustrating one example of using a guide collar.

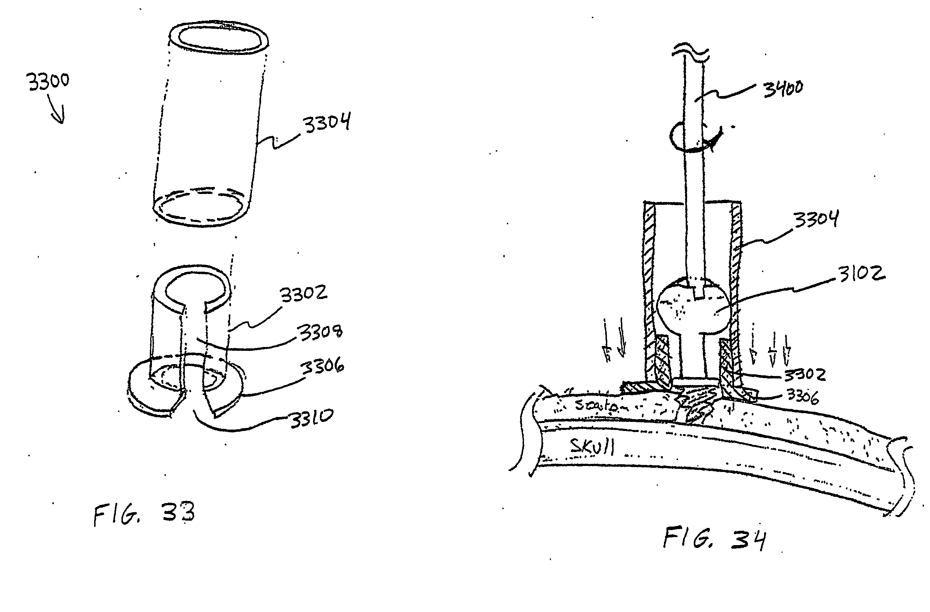

FIG. **33** is a perspective view of an alternative guide collar.
FIG. **34** is a side sectional view of a guide base, a height extender, a fiducial marker, and a screwdriver shaft.

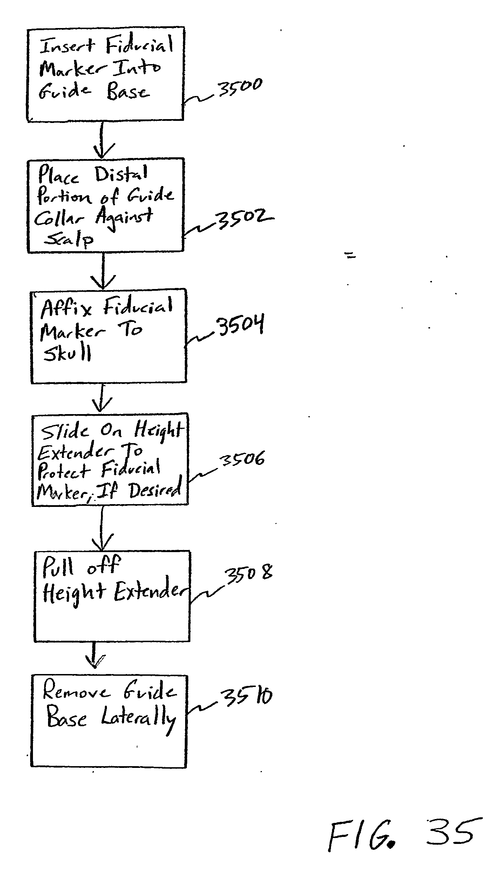

FIG. **35** is a flow chart illustrating one example of using a guide base and a height extender.

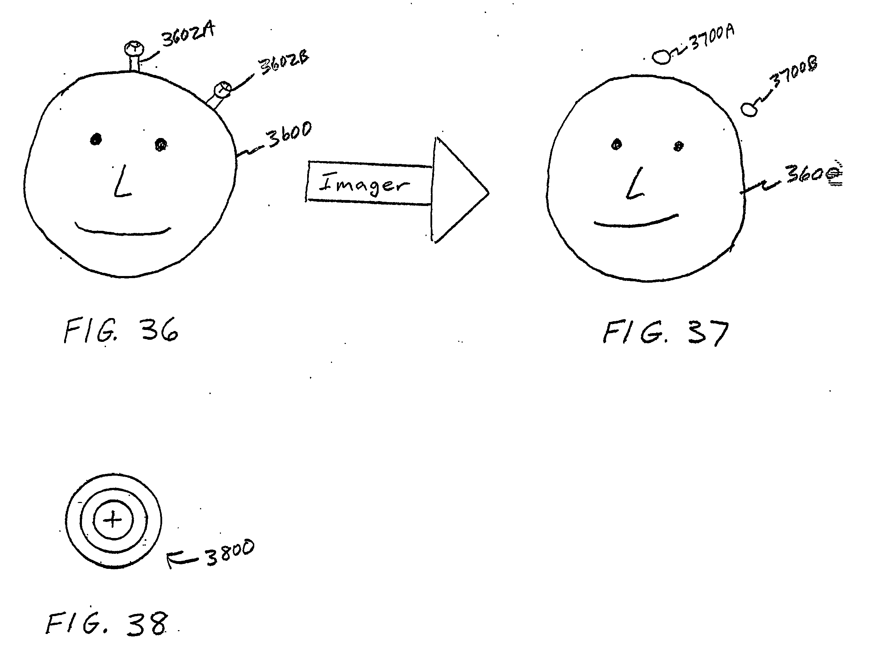

FIG. **36** is a schematic illustration of a subject with one or more fiducial markers affixed to the subject's skull.
FIG. **37** illustrates schematically one example of how such fiducial marker head images appear on an image.
FIG. **38** illustrates schematically one example of a template, such as for assisting a user in locating respective centers of fiducial marker head images.

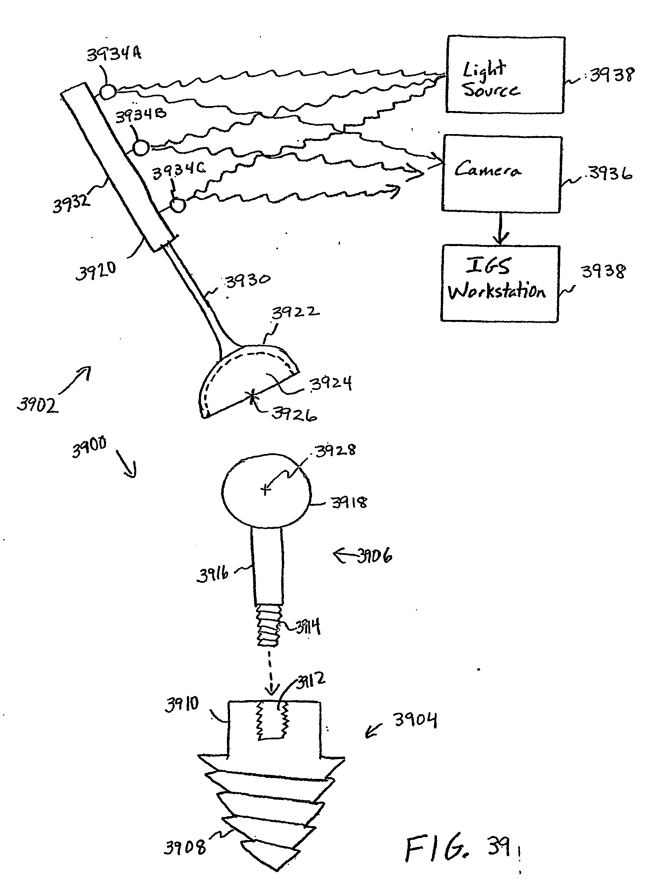

FIG. **39** is a schematic diagram illustrating generally one example of a fiducial marker and a positioning system including a positioning wand with a semispherical cap.

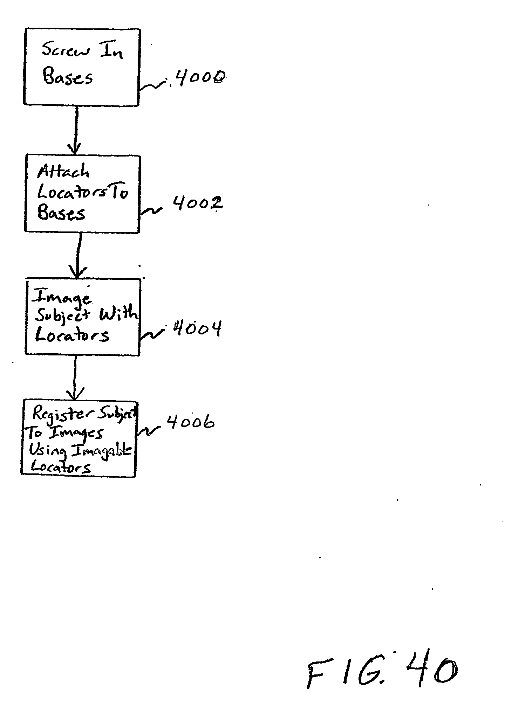

FIG. **40** is a flow chart illustrating generally one example of using devices such as are shown in the example of FIG. **39****.**

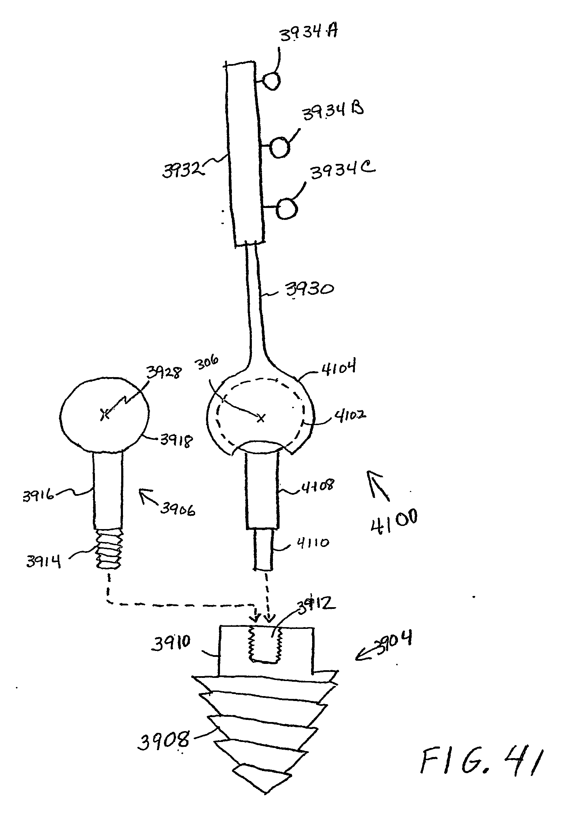

FIG. **41** is a schematic diagram illustrating generally an alternative example of a positioning wand that includes a ball and a socket or other joint.

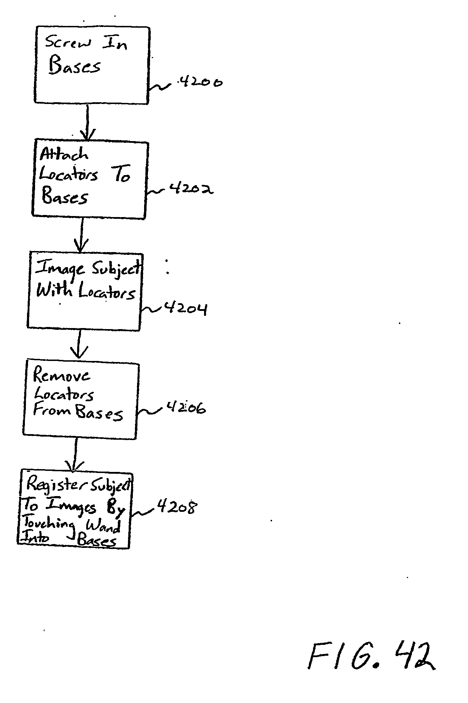

FIG. **42** is a flow chart illustrating generally one example of using the devices illustrated in FIG. **41** and FIG. **39****.**

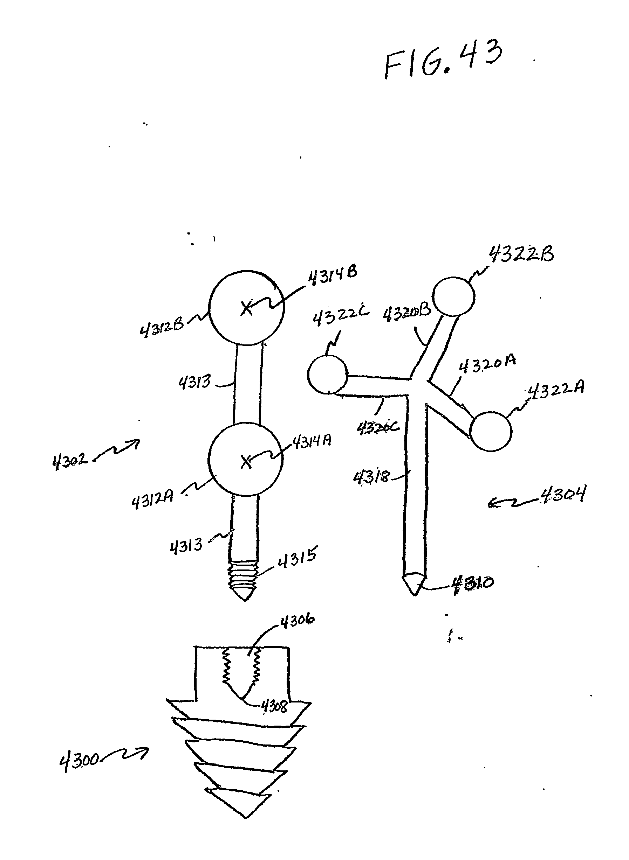

FIG. **43** is a schematic diagram illustrating generally an alternative example of a locator with two imageable spheres and a base with a built-in registration receptacle.

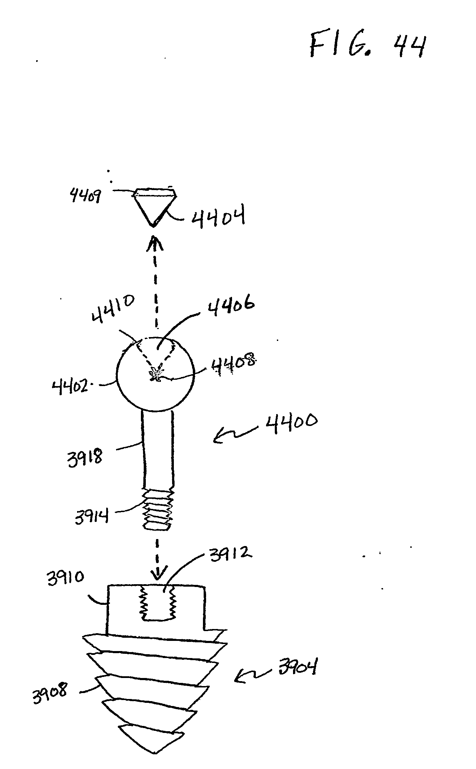

FIG. **44** is a schematic diagram illustrating generally an alternative example of another locator, with an imageable sphere that includes a removable imageable cone.

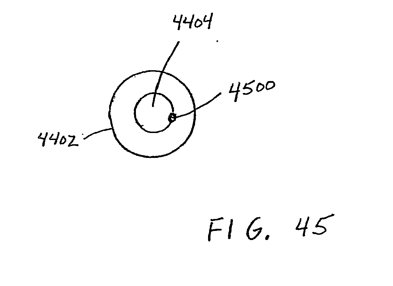

FIG. **45** is a schematic diagram illustrating generally a top view of the imageable sphere and included imageable cone of FIG. **44****.**

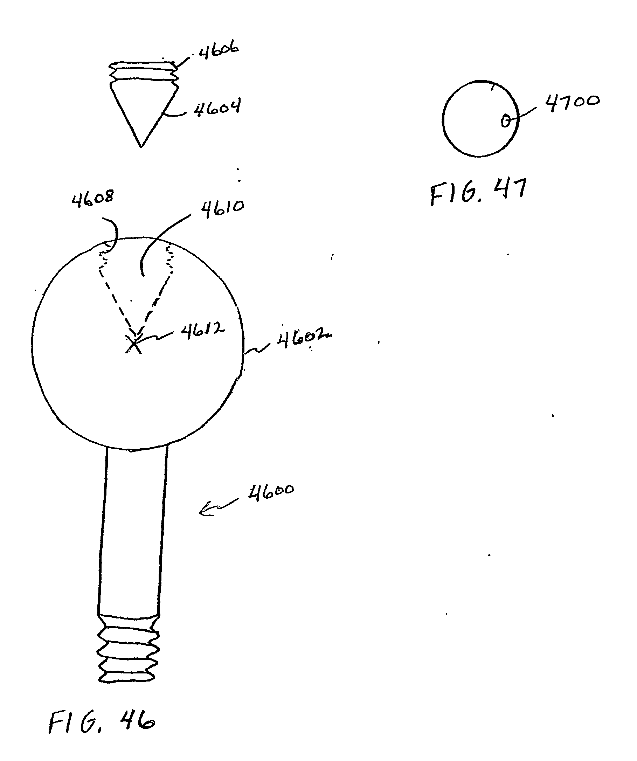

FIG. **46** is a schematic diagram illustrating generally an alternative example of a locator including an imageable sphere with a removable imageable cone.
FIG. **47** illustrates a top view of the cone of FIG. **46****.**

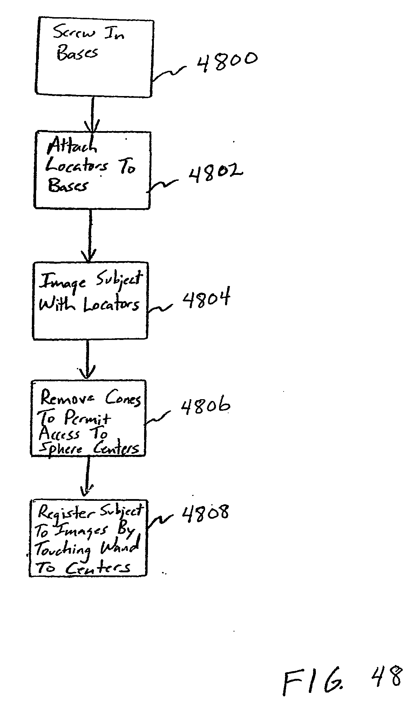

FIG. **48** is a flow chart illustrating generally one example of a method for using the devices illustrated in FIGS. **44 - 47****.**

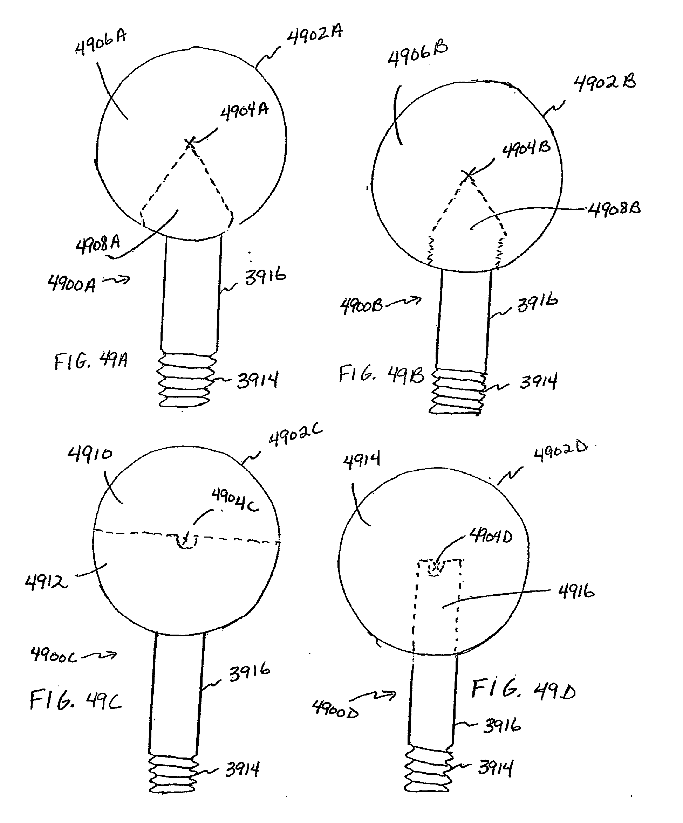

FIG **49A,** FIG. **49B**, FIG. **49C,** and FIG. **49D** are schematic diagrams illustrating generally other examples of locators having imageable spheres that include removable imageable components.

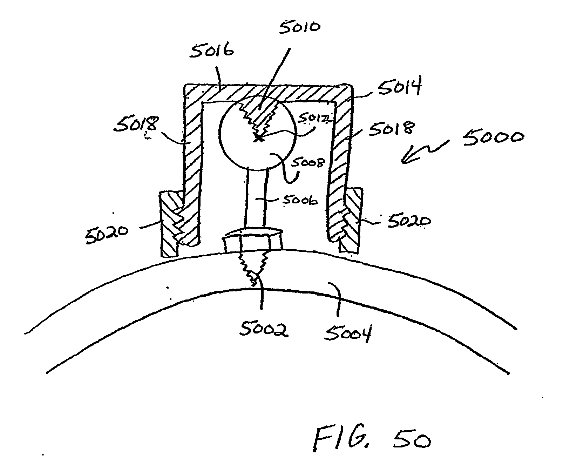

FIG. **50** is a schematic diagram illustrating generally an example of a fiducial marker assembly.

### DETAILED DESCRIPTION

In the following detailed description, reference is made to the accompanying drawings which form a part hereof, and in which is shown by way of Illustration specific devices, methods and systems.. It is to be understood that embodiments may be combined, or that other embodiments may be utilized and that structural, logical and electrical changes may be made. The following detailed description is, therefore, not to be taken in a limiting sense, and the scope of the present invention is defined by the appended claims

In this document, the terms "a" or "an" are used, as is common in patent documents, to include one or more than one.
In this document, the term "assembly" is not intended to be limited to a structure that is assembled from multiple components, but also includes unitary or integrally-formed structures or the like.

### Example 1

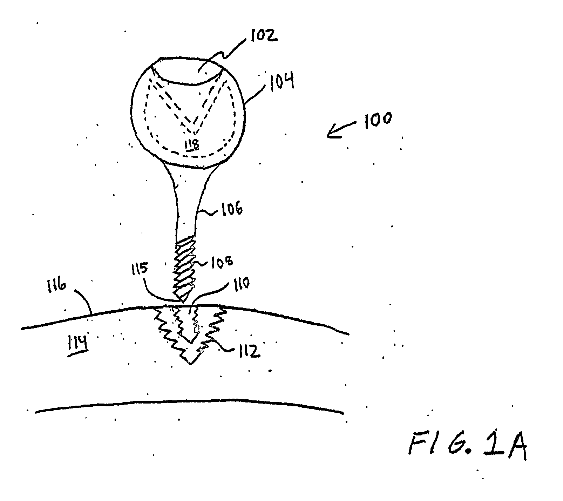

Figure 1A is a schematic diagram illustrating generally, by way of example, but not by way of limitation, one example of an imagable fiducial marker 100 that includes a built-in divot 102. In this example, the divot 102 includes a female receptacle, such as the illustrated conical depression. However, as used herein, a divot also refers to any other male or female receptacle, or the like. The divot 102 is capable of receiving a correspondingly sized and shaped mating tip of a positioning wand or like instrument. Such a wand or instrument is useful for registering the actual physical location of the patient's skull to preoperative or other images of the subject's brain. Such images are typically stored in a memory of an image-guided surgical (IGS) computer workstation.

In the example illustrated in Figure **1A****,** the fiducial marker **100** includes an imagable substantially spherical fiducial locator **104.** The fiducial **104** is locatable using one or more imaging system modalities. In this example, a shaft **106** extends orthogonally outward from a circumferential portion of the spherical fiducial **104.** The shaft **106** includes an externally threaded portion **108.** The externally threaded portion **108** is sized and shaped for being received within a correspondingly sized and shaped mating internally threaded receptacle **110** of an externally-threaded self-tapping base **112.** In this example, the base **112** is capable of being mounted in a skull **114,** such as either flush to (or even recessed from) an outer surface **116** of the skull **114.** One example of a suitable base **112** is described in commonly-assigned Mazzocchi et al. U.S. Patent Application Serial No. 10/206,884 entitled FIDUCIAL MARKER DEVICES, TOOLS, AND METHODS, which was filed on July 24, 2002, including its disclosure relating to a flush or recessed mounted base and other fiducial marker devices, tools and methods. However, in alternative examples, the base **112** need not be configured for mounting flush to or recessed from the outer surface **116** of the skull **114.** In this example, the shaft **106** includes a pointed tip **115.** This permits the shaft **106** to more easily penetrate a sterile drape that, in certain circumstances, may be placed over the patient's skull **114.** Moreover, in this example, the receptacle **110** of the base **112** is shaped to accommodate the pointed tip **115.** However, in an alternative example, the tip **115** need not be pointed.

In one example, the imaging spherical fiducial locator **104** houses a generally spherical (e.g., except for the conic cutaway of the divot **102)** sealed interior cavity **118.** In one example, the cavity **118** is filled with an imagable fluid that is visible on one or more imaging modalities (e.g., MR, CT, etc.). In this example, the apex of the conic divot **102** is located at a spherical center of mass of the imaging spherical fiducial locator **104** (i.e., the apex is located where the center of mass would be if the imaging fiducial locator **104** were perfectly spherical, without any cutout divot). This allows the tip of a positioning wand (recognizable by a camera in an optical position locating system that is coupled to the image-guided surgical workstation) to be inserted into the divot **102.** This results in the wand tip being located at the spherical center of mass of the imaging spherical fiducial locator **104.** This is useful for assisting in registering the physical location of the patient to the preoperative images stored in the image-guided surgical workstation.

Unlike fiducial marker assemblies that require the user to attach an imaging fiducial while obtaining the preoperative images of the patient's brain, and to then replace that imaging fiducial with a separate divot during patient registration in the operating room, the fiducial marker **100** illustrated in Figure **1A** does not require any such exchange of the imaging fiducial for a separate divot. Instead, the divot is integrated into the imaging fiducial itself, as illustrated in Figure **1A****.** This reduces the complexity of the image-guided surgical procedure and, therefore, reduces its cost. It also reduces the complexity of manufacturing, which, in turn, reduces manufacturing costs.

In one example (but not by way of limitation), the base **112** is constructed of stainless steel. The shaft **106** and the imaging spherical fiducial locator **104** are constructed of molded plastic polymer. In this example, the imaging spherical fiducial locator **104** includes an open cavity **113** for receiving the imaging fluid, and for then receiving an insertable plastic conical divot **102** that adhesively or otherwise seals the cavity **118** to retain the imaging fluid therein. The imaging fluid in the cavity **118** is visible and provides good contrast on images produced by at least one imaging modality. In one example, the imaging fluid is multimodal (i.e., locatable by more than one imaging modality), such as by using a mixture of different imaging fluids that are locatable on different imaging modalities. In an alternative example, the plastic forming the imaging spherical fiducial locator **104** includes a substance that is viewable on a first imaging modality, while the imaging fluid within the cavity **118** is viewable on a different second imaging modality.

In one such illustrative example, the plastic imaging fiducial locator **104** is doped with a substance having a high atomic number (Z), such as barium, titanium, iodine, silver, gold, platinum, iodine, stainless steel, titanium dioxide, etc. that provide good contrast on a CT or other radiographic imaging system. In this illustrative example, the fluid within the cavity **118** includes gadopentatate dimeglumine, gadoteridol, ferric chloride, copper sulfate, or any other suitable MRI contrast agent, such as described in chapter 14 of Magnetic Resonance Imaging, 2nd ed., edited by Stark and Bradley, 1992, which is incorporated herein by reference.

In an alternative multimodal example, the cavity **118** is omitted. Instead, the spherical fiducial locator **104** is constructed of a substantially solid plastic or other material that is hygroscopic, that is, capable of receiving and retaining a fluid, such as an imaging fluid that is viewable on an imaging system (e.g., an MRI imaging system or the like). In a further example, the plastic forming the spherical fiducial locator **104** is doped or otherwise includes a substance that is viewable on a different imaging system, such as, for example, a CT or other radiographic imaging system. Illustrative examples of solid plastics that can be made hygroscopic include, among other things, nylon and polyurethane. Using a hygroscopic material avoids the complexity and cost associated with manufacturing a sealed cavity **118** for retaining an imaging fluid. Moreover, by adapting the solid hygroscopic plastic for imaging using a first modality, and by using the imaging fluid for imaging using a second modality, each of the solid and the fluid can be separately tailored toward providing better contrast for its particular imaging modality.

In another alternative example in which the cavity **118** is omitted, the fiducial locator **104** includes a rigid solid (e.g., substantially spherical, but for the conic divot) interior. This solid material is doped with a substance that provides good contrast using a first imaging modality (e.g., CT). A hygroscopic outer coating is formed thereupon. The coating permits soaking up a fluid that provides a good contrast using a second imaging modality (e.g., MRI).

In a further example of the fiducial marker **100** illustrated in Figure **1A****,** the outer surface of the imaging spherical fiducial locator **104** is reflective of light or other electromagnetic energy. Consequently, it is also locatable by the operating room camera in an optical positioning system that is coupled to the image-guided workstation (e.g., during patient registration). In one such example, the outer surface of the imaging spherical fiducial locator **104** includes light-reflective micro-spheres (e.g., embedded in an adhesive covering the imaging spherical fiducial **104**). In another such example, the outer surface of the imaging spherical fiducial **104** is covered with an adhesive-backed light-reflective tape, such as SCOTCHLITE^{®} 9810 Reflective Material Multipurpose Tape sold by Minnesota Mining and Manufacturing Co. ("3M^{®}"), of Saint Paul, Minnesota.

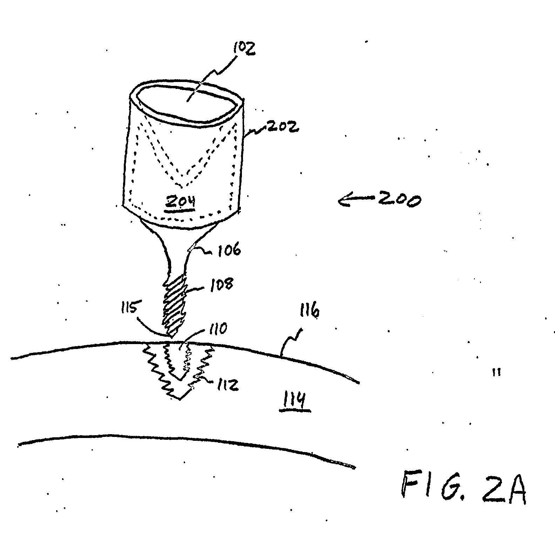

Figure **2A** is a schematic diagram illustrating generally, by way of example, but not by way of limitation, an alternative example of a fiducial marker **200** that includes a generally cylindrical imaging fiducial locator **202** and a conical or other divot **102.** In one example, the generally cylindrical imaging fiducial locator **202** includes a sealed cavity **204** for receiving and retaining an imagable fluid, as discussed above. In another example, the sealed cavity **204** is omitted, as discussed above. In one such example, the generally cylindrical imaging fiducial locator **202** is instead constructed of a substantially solid hygroscopic plastic that carries an imagable fluid (as discussed above), such as for providing multimodal contrast across different imaging modalities. In a further example, the generally cylindrical outer surface of the imaging fiducial locator **202** is reflective, as discussed above, such that the imaging fiducial locator **202** is also visible to a camera of an optical position locating system that is coupled to an image-guided surgical workstation (e.g., during patient registration and/or a subsequent image-guided surgical procedure). In one such example, the imaging fiducial locator **202** is covered with adhesive-backed reflective tape taken from a rectangular strip of such tape that is wound into a roll. In this example, the generally cylindrical shape of the outer surface of the imaging fiducial locator **202** is much easier to wrap using a wound rectangular strip of the adhesive reflective tape than a spherical surface, such as is illustrated in Figure **1A****,** and therefore costs less to manufacture. In this document, the term "generally cylindrical" is not limited to a perfectly cylindrical surface, but instead is understood to include any faceted or other column or like structure (e.g., an octogonal cylinder a hexagonal cylinder, etc.) that includes a lateral peripheral surface that easily accommodates receiving a wound rectangular or similar strip of tape (as opposed to a spherical, elliptical, or conical surface, to which is more difficult to evenly apply a wound rectangular strip of tape taken from a roll). Examples of such generally "cylindrical" columnar structures having faceted lateral peripheral surfaces are illustrated in Figure **3C****.**

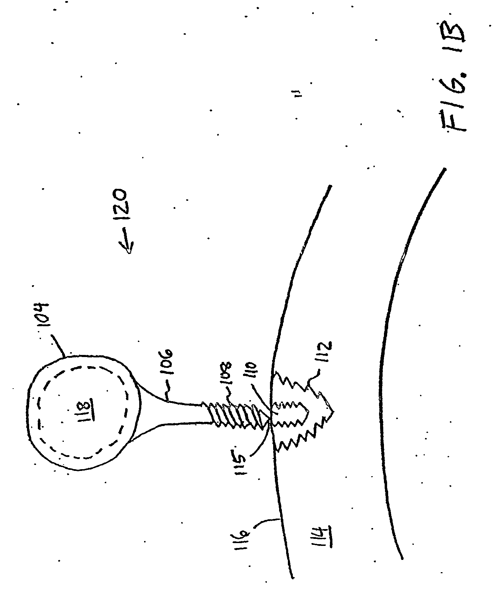

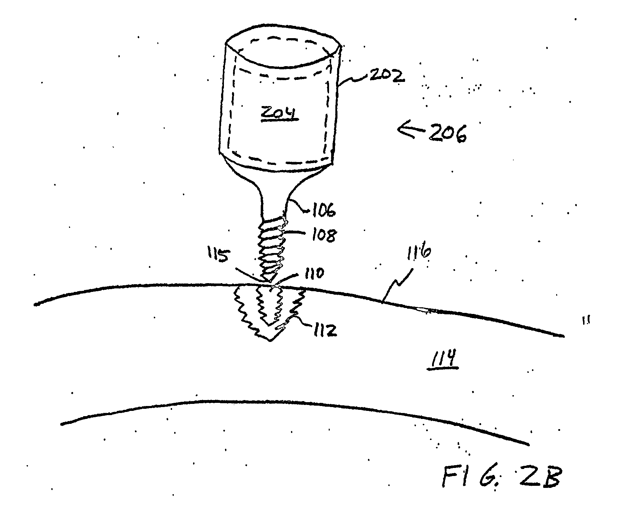

In an alternate example to the illustrations of Figures **1A** and **2A****,** the divot **102** is omitted from the fiducial marker **100** or **200.** However, the resulting fiducial marker is still configured to be locatable by a remote positioning system as well as imagable using one or more imaging modalities. In one such example, the outer surface **104** or **202** is still configured to be light reflective, such as discussed above. In one such example, the fiducial markers **100** and **200** still advantageously are locatable using one or more imaging modalities (e.g., MR, CT, or other imaging system providing 3D or other internal images within a subject) as well as also being locatable external to the subject, such as by using a remote camera or like component of an optical or other positioning system, e.g., that is coupled to an image-guided workstation. In one example, this permits automatic registration of the actual location of the subject in the operating room (e.g., using the cameras to locate the light reflective fiducial markers **100** or **200)** to preoperative images of the patient on which the same imagable fiducial markers **100** and **200** appear. This eliminates any need to register the patient by inserting an optically-locatable positioning wand tip into a divot of each fiducial marker (and also eliminates any need for a reference divot or other absolute position reference), because the fiducial markers themselves are optically locatable and registerable to known locations on the preoperative images. Therefore, in this example, the divots **102** are not needed and can be omitted, as illustrated by the divotless spherical imagable reflective fiducial marker **120** in Figure **1B** and the divotless cylindrical imagable reflective fiducial marker **206** in Figure **2B****.** Although Figure **2B** illustrates an example including a cavity **204** for carrying a liquid contrast agent, in an alternative example, the cavity **204** is omitted, and the fiducial marker **206** includes a solid structure that is doped or otherwise configured (e.g., hygroscopic) for providing good imaging contrast using one (e.g., CT) or more imaging modalities.

In yet another example, the fiducial markers **100** and **200** respectively illustrated in Figures **1A** and **2A** include the illustrated divots **102** and are locatable by a remote positioning system (such as by including light-reflective outer surfaces and/or embedded coils that perform magnetic field sensing in a magnetic field based positioning system). However, in this example, the fiducial markers **100** and **200** need not be configured for providing contrast on the one or more imaging modalities. In such an example, the preoperative images are taken with imagable fiducial markers placed within respective bases **112.** Such imagable fiducial markers are then replaced (within their respective bases **112**) by nonimagable fiducial markers that are locatable by a remote positioning system, such as by including both a divot and a light-reflective surface. The light reflective surface permits automatic location by the remote positioning system. However, if the reflective surface is dirty or otherwise unrecognizable by the remote positioning system, a wand or other locating instrument can be placed within the divot to perform the remote locating of the fiducial marker.

Moreover, although Figures **1A** and **2A** illustrate examples in which a shaft **106** is received within a base **112** that is mounted flush to (or recessed from) the outer surface **116** of the skull **114,** this is not required. In one alternate example, the shaft **106** is manufactured as a stainless steel or other suitable material that is capable of acting as a self-tapping bone screw. In such an example, the threaded portion **108** of the shaft **106** is threaded directly into the skull **114** without using any base **112.** In another alternate example, the base **112** includes a shaft or flange portion that rises above the outer surface **116** of the skull **114.** In certain examples, the fiducial markers **100** and **200** may use a threaded or other shaft **106** for coupling to the base **112,** or alternatively may use a snap-fit clip or a like attachment device for coupling to the base **112.**

Figure **3A** is a schematic diagram illustrating generally, by way of example, but not by way of limitation, one example of a positioning wand **300,** such as for use with a remotely-located camera or other like device of an optical positioning system configured for being coupled to an image-guided surgical workstation in an operating room. In this example, the wand **300** includes a tip **302** that is sized and shaped to permit being received in a divot **102** of a skull-mounted fiducial marker (such as fiducial markers **100** and **200).** The wand **300** includes a plurality of cylindrically-shaped fiducial locators **304** that are locatable by the camera or other like device of the optical positioning system. The fiducial locators **304** (which typically need not include divots) on the wand **300** are positioned in a known spatial relationship to each other and to the tip **302** of the wand **300.** By recognizing the locations of the fiducial locators **304,** the optical positioning system is capable of computing the location of the wand tip **302,** which is in a known spatial relationship with the configuration of fiducial locators **304.** This permits the wand **300** to be used in conjunction with the optical positioning system to register the patient and to further plan and/or perform the surgical procedure using the image-guided surgical workstation. The fiducial locators **304** are covered with adhesive-backed reflective tape, as discussed above. The cylindrical (or faceted cylindrical) shape of the fiducial locators **304** permits easier wrapping by the reflective tape than the spherical fiducials, as discussed above. This reduces the cost of manufacturing the fiducial locators **304** and, in turn, reduces the cost of manufacturing the positioning wand **300.**

Figure **3B** is a schematic diagram, similar in certain respects to Figure **3A****,** but illustrating a wand **306** that includes locators **308A-C** having swiveling or fixed cylindrical locators **308A-C** having respective slanted (e.g., flat, parabolic, or other) top surfaces **310A-C** (e.g., non-orthogonal with respect to a longitudinal center axis **311** of the locator **308)** that reflect light or other electromagnetic energy for being located by a remote detector. In an example in which the locators **308A-C** swivel, each such locator **308** includes a shaft inserted into a hole or other receptacle in the wand **306.** This permits the locator **308** to rotate with respect to its mounting location on the wand **306.** Either the wand **306** itself or the individual locators **308A-C** are oriented by the user to aim the reflective surfaces **310A-C** toward a camera or other detector of an optical positioning system. In one further example, the circumferential surfaces of the cylindrical locators **308A-C** are also light-reflective, however, this is not required. In one such cost-effective example, the reflective tape disks are adhered to the flat slanted top surfaces **310A-C** and the circumferential lateral surfaces of the cylindrical locators **303A-C** are not reflective.

Figure **3C** is a perspective view schematic diagram illustrating generally, by way of example, but not by way of limitation, certain generally "cylindrical" columnar structures **312, 314,** and **316** having faceted lateral peripheral surfaces. Such surfaces are conducive to receiving a rectangular or like strip of adhesive reflective tape. Such structures, therefore, are particularly well-suited for implementing locators that are remotely locatable by an optical positioning system. Such remotely detectable locators are suitable for use in the fiducial markers illustrated in Figures **2A** and **2B****,** as well as for use in the remotely detectable locators of the positioning wands illustrated in Figures **3A** and **3B****.** Such remotely detectable locators are also useful for being affixed in a known relationship to the patient, such as to the operating table or to a skull-immobilizing headframe. This provides a remotely detectable absolute positional reference to an optical positioning system. Such remotely detectable locators are also useful for being affixed to a biopsy needle, shunt catheter, or other instrument being introduced through a trajectory guide device or otherwise used in an image-guided surgical procedure.

Figure **3D** is a schematic diagram illustrating generally, by way of example, but not by way of limitation, an alternative example of a positioning wand **318.** In this example, which flat disk-shaped pieces of reflective tape are attached to the wand **318** in a known configuration, such as at the distal ends of radial arms extending therefrom.

### Example 2

Figure **4** is a schematic diagram illustrating generally, by way of example, but not by way of limitation, an image guided surgical (IGS) computer workstation **400,** which is capable of displaying previously acquired and loaded preoperative images of a patient's skull. On these preoperative images appear viewable images of imagable fiducial markers that were screwed into the patient's skull before the preoperative imaging (e.g., using MRI, CT, etc.). In the example illustrated in Figure **4****,** the imagable fiducial locators have been unscrewed from respective bases **402** screwed into the patient's skull. The imagable fiducial locators have been replaced by patient registration divot assemblies **404** that have been screwed into (or otherwise coupled to) respective bases **402** in the patient's skull **114.** In this example, the registration divot assemblies **404** are configured to receive a shaft tip **406** of a positioning wand **408** that is locatable by one or more remote cameras **410A-B** (or other sensing devices) of an optical position detection system **412** connected to the IGS workstation **400.** In one example, the positioning wand **408** includes spherical reflective fiducial locators **414.** The fiducial locators **414** are arranged in a known spatial relationship to each other (however, it may alternatively use other reflective locators such as discussed elsewhere in this document). The optical positioning system **412** includes an infrared light (or other energy source) **416** that provides light that is reflected from the reflective fiducial locators **414.** This permits the reflective fiducial locators **414** on the positioning wand **408** to be located and recognized by the cameras **410A-B.** In some circumstances, however, the field of view (or "sweet spot" of the field of view) provided by cameras **410A-B** is limited. This sometimes makes it difficult for the optical positioning system **412** to recognize the positioning wand **408.** Moreover, the recessed receptacle in the divot assembly **404** typically limits the range within which the probe **408** can be manipulated (e.g., to bring it within the field of view) while retaining the wand tip **406** within the recessed receptacle.

Figure **5** is a schematic diagram illustrating generally, by way of example, but not by way of limitation, a unitary divot assembly **500** that includes multiple divots **502.** In this example, the unitary divot assembly **500** is configured such that it can be threaded into or otherwise coupled to a base **504** that is secured to the patient's anatomy (wherein the base **504** is also configured for alternatively receiving an imagable fiducial locator, e.g., during preoperative imaging). Figure **5** illustrates multiple conical receptacle divots **502** having commonly located apexes. These commonly located apexes are designed to coincide with the center of the image produced by the imagable fiducial locator for which the divot assembly **500** has been substituted during patient registration. In the illustrated example, the divots include a top conical divot **502A** and four side conical divots **5028-F.** The four side conical divots **502B-F'** are distributed around the cylindrical lateral peripheral circumference of the upper portion of the divot assembly **500.** The wand tip **406** may be inserted into any one of the divots **502.** This permits a greater range of motion of the positioning wand **408.** As a result, it is easier to bring the reflective fiducials **414** on the positioning wand **403** into the field of view of the cameras **410A-B** of the optical positioning system **412.**

Figure **6A** is a schematic diagram illustrating generally, by way of example, but not by way of limitation, a divot assembly **600** that includes a swiveling tilted head **602** carrying a conical or other divot **604** or the like. In this example, the head **602** is tilted with respect to a cylindrical coupling **606** extending outwardly therefrom. The coupling **606** includes a hollow interior or other (female or male) connector that snap-fits onto and rotatably rides upon a mating (male or female) connector **608** that is located at a proximal end of a shaft **610** portion of the divot assembly **600.** The swiveling apex **612** of the divot **604** is designed to coincide with the center of mass of the imagable fiducial locator for which the divot assembly **600** has been substituted during patient registration. The swiveling tilted head **602** permits a wide range of motion of the positioning wand **408** when the wand tip **406** is inserted into the divot **604.** As a result of such rotational articulation, it is easier to bring the reflective fiducial locators **414** on the positioning wand **408** into the limited field of view of the cameras **410A-B** of the optical positioning system **412.**

Figure **7A** is a schematic diagram illustrating generally, by way of example, but not by way of limitation, a divot assembly **700** that includes a swiveling and pivotable head **702** carrying a conical or other divot **704.** In this example, the head **702** is carried by a shackle-like U-shaped bracket **704** that rotatably rides upon a snap-fit or other capturing post **706** that extends upward from a shaft portion **708** of the divot assembly **700.** This allows swiveling of the bracket **704** (and the head **702** carried by the bracket **702**) with respect to the shaft **708.** In this example, the head **702** is suspended between upward-projecting risers of the bracket **704** by axels **710A-B** extending outward from opposing sides of the head **702** and received within corresponding receptacles in the risers of the bracket **704.** This permits pivoting/tilting articulation of the head **702** with respect to the swiveling bracket **704.** Therefore, this example provides a swiveling and adjustably tiltable divot **704** that is designed such that its apex **712** coincides with the center of mass of the imagable fiducial locator for which the divot assembly **700** has been substituted during patient registration. Among other things, the swiveling tiltable head **702** advantageously permits a greater range of motion of the positioning wand **408** when the wand tip **406** is inserted into the divot **704.** As a result, it is easier to bring the reflective fiducials **414** on the positioning wand **408** into the limited field of view of the cameras **410A-B** of the optical positioning system **412.**

Figures **6B** and **7B** are schematic diagrams that are similar in certain respects to Figures **6A** and **7A****.** However, the locator assemblies **614** and **714** illustrated by respective Figures **6B** and **7B** omit the respective divots **604** and **704.** Instead, the locator assemblies **614** and **714** provide aimable electromagnetic energy (e.g., light) reflective surfaces **616** and **716,** respectively. The reflective surfaces **616** and **716** are aimed at the camera of an optical positioning system **412** to allow automatic detection of the locator assemblies **614** and **714** without requiring the use of a positioning wand **408.**

. The reflective surfaces **616** and **716** are configured so that, when aimed properly, they produce a reflected image that can be correlated to a previously acquired patient image on which an image of an imagable fiducial marker appears. In one such example, reflective surface **616** corresponds to the center of mass of a similarly sized spherical locator on an imagable fiducial marker assembly for which locator assembly **614** is substituted during patient registration. In another such example, reflective surface **716** includes a circular disk-shaped piece of reflective tape affixed to a surface **718** such that this reflective disk pivots about the axis provided by axels **710A-B.** In this manner, the reflected disk shape corresponds to the center of mass of a similarly sized spherical locator on an imagable fiducial marker assembly for which locator assembly **714** is substituted during patient registration.

### Example 3

As discussed above, screwing multiple fiducial markers into different locations in the patient's **skull 114** results in trauma and/or risk of infection at each one of such multiple different locations. Figure **8** is a schematic diagram illustrating conceptually, by way of example, but not by way of limitation, a fiducial marker carrier **800** that is attachable to (and also detachable from) a single location on the patient's skull **114,** thereby reducing trauma and risk of infection to the patient. In this example, the fiducial marker carrier **800** is configured for carrying multiple different imagable fiducial locators **802** such that they are positioned at different locations about the patient's skull **114.** As discussed below, the carrier **800** uses a keyed mounting arrangement, such that the carrier **800** can be attached to the patient's skull **114,** then detached from the patient's skull **114,** and later reattached to the patient's skull **114** in the same orientation in which it was initially attached to the patient's skull **114.**

In the example illustrated in Figure **8****,** the carrier **800** includes a keyed frame **804** that is attached to a keyed post **806** for mounting. The keyed post **806** is, in turn, attached to a single flush-mounted or recessed-mounted or other keyed base **808,** which was previously screwed into the patient's skull **114.** This keyed arrangement of the frame **804,** the post **806,** and the base **808** permits attachment, detachment, and reattachment in the same orientation as the original attachment, as discussed above. In an alternative example, the post **806** is integrally formed as part of the frame **804,** rather than being keyed for attachment thereto.

In one example, such illustrated in Figure **8****,** the imagable locators **802** are placed about the subject's head such that they surround the patient's skull. Although such a surrounding arrangement is not required, it is believed to improve the accuracy of using the images of the locators **802** (e.g., in conjunction with the IGS workstation) for planning and/or performing an image-guided surgical procedure, as compared to an arrangements in which locators are disposed more closely together (e.g., on the same side of the subject's head).

Figure **9** is an exploded view schematic diagram illustrating generally, by way of example, but not by way of limitation, one example of the carrier **800,** including the frame **804,** the post **806,** and the base **808.** In this example, the ° base **808** includes self-tapping external threads **902,** and is capable of being mounted flush with (or even recessed within) the patient's skull **114.** The base **808** includes an internally-threaded receptacle **904** that is sized and otherwise configured such that it is capable of receiving a screw. The base **808** also includes a female or male keying feature for receiving a mating keying feature of the post **806** to fixedly define the orientation of the post **806** with respect to the base **808.** In one example, the keying feature includes a key slot **906** extending radially outward from the receptacle **904** along a proximal surface of the base **808.**

The post **806** includes a proximal end **908** and a distal end **910.** The post 806 includes a center lumen **912** in which an attachment screw **914** is received and seated. The screw **914** attaches the post **806** to the base **303.** The distal end **910** of the post **806** includes a male or female keying feature (such as a key protrusion **916** extending radially outward from the center lumen **912** along the distal end **910** of the post **806**) that mates with the keying feature (e.g., key slot **906**) of the base **808.** Such mating during the attachment fixedly defines the orientation of the post **806** with respect to the base **808.**

In this example, the center lumen **912** includes a keyed seating receptacle **918** (or an analogous male keyed feature) for receiving a mating keyed feature of the frame **804.** In the illustrated example of Figure **9****,** the keyed seating receptacle **918** includes an increased diameter of the center lumen **912** (with respect to more distal portions of the center lumen **912**) to provide the seating, and a radially-outwardly extending slot **920** to provide the keying.

In the example illustrated in Figure 9, the frame 804 includes legs 922A-D (or a fewer or greater number of legs 922), such as extending radially outwardly from a hub 924 and downwardly toward the middle portion of the patient's skull. Each of the legs 922 Includes, such as at its respective distal end, a threaded receptacle 924A-D (or a snap-fitting or any other coupling) for receiving at least one of an imageable fiducial marker assembly 926, a pivot assembly 928, a locator assembly 930 (e. g., reflector, LED, microcoil, etc.) that is remotely detectable by a positioning system in an operating room, or a combination 932 of two or more of the above. In an alternative example (for example where a combination 932 includes an imagable locator and at least one of an operating room position locator and a divot), instances of such a combination 932 may be permanently affixed to corresponding locations on the legs 922 of the frame 804.

In the example illustrated in Figure 9, the hub 924 portion of the frame 804 also includes a downwardly protruding key 934 (or analogous female receptacle) that mates to the keyed seating receptacle 918, of the post 806, into which the key 934 is received. This fixedly defines the orientation of the frame 904 with respect to the post 806. A screw 936 is inserted through the hub 924, the key 934, and into an engaging interior threaded portion of the center lumen 912. This securely attaches the frame 904 to the post 806 in the fixedly defined orientation. The example illustrated in Figure 9 also includes at least one optional instrument mount 938. In one example, a reference divot (e.g., providing a position reference) is attached to the instrument mount 938.

Although Figures 8 and 9 illustrate examples in which a fiducial marker carrier 800 is mounted using a single base 808, in other examples, the carrier may be mounted using two or more bases 808 at the same location on the patient's skull (that is, at adjacent locations within the same scalp incision, or like limited trauma/infection risk zone; the incision need only be large enough to accommodate the two or more bases 808). Using two or more side-by-side bases 808 to attach the post 806 avoids potential rotational misalignment of a single base 808 coming slightly unscrewed from its original position.

Alternatively, if a single base 808 is used, such rotational misalignment can be avoided by including one or more antirotation spikes 1000 on the bottom of the distal end 910 of the post 806, such as illustrated generally in Figure 10.
In the example illustrated in Figure **10****,** the distal end **910** of the post **806** is keyed both to the base **808** and, using the antirotation spike(s) **1000,** to indentation(s) made in the surface **116** of the skull **114.** However, in an alternative example, the post **806** and the base **808** need not be keyed to each other. Instead, in such an example, the post **806** is keyed only to indentation(s) made by the antirotation spike(s) **1000** in the surface **116** of the skull **114.**

### Example 4

FIG. **11** is a perspective view of an alternative example of a fiducial marker **1100.** FIG. **12** is a top view of the fiducial marker **1100** illustrated in FIG. **11****.** In the example of FIGS. **11-12****,** the unitary fiducial marker **1100** includes a substantially spherical head **1102.** A unitary fiducial marker includes both a single piece as well as multiple pieces that are assembled into a single assembly that, in use, is not disassembled or otherwise decomposed into more than one separate component. In this example, a divot **1104** is cut out from a proximal portion of the head **1102.** The divot **1104** is shaped to receive a corresponding mating shaped portion of a remote positioning locator. In one illustrative example, the divot **1104** is conical (as illustrated in FIG. **11**), such as to receive a mating conical tip **302** of the positioning wand **300** illustrated in FIG. **3****,** or a similar probe tip. An apex of the inverted conical divot **1104** corresponds to a centroid of the substantially spherical head **1102.** In this example, a bone screw shaft **1106** extends outward from an opposite (e.g., distal) portion of the head **1102.** (Alternatively, if a sterile drape or the like is to be used between the tip **302** of the wand **300** and the divot **1104** of the fiducial marker **1100,** then, in one example, the location of the apex of the divot **1104** may be adjusted to offset the thickness of the sterile drape such that the tip **302** of the wand **300** is located at the centroid of the head **1102** even when the drape is interposed between the tip **302** and the divot **1104**).

In this example, the conical divot **1104** of the head **1102** includes slots **1108** extending therefrom. The slots **1108** accommodate a driving tip of a screwdriver (e.g., Phillips and/or flathead, etc.). In this manner, the slots **1108** permit the fiducial marker **1100** to be screwed into a skull, bone, or other structure. Alternatively, the divot **1104** includes any other known rotational engagement structure for permitting rotation of the fiducial marker **1100** for threading it into bone, as discussed below.

In one example, the shaft **1106** includes one or more self-tapping or other external bone screw threads **1110,** which are sized and shaped for being threaded into bone, such as a patient's skull. In one example, a distal tip of the shaft **1106** includes at least one cutout, such as a quarter cylindrical cutout **1112.** In this example, the vertically-oriented flute-like cutout **1112** portion of the shaft **1106** assists in cutting bone as the shaft **1106** is being turned for threading into the bone. The self-drilling cutout **1112** and self-tapping nature of the threads **1110** are not essential. These features are not needed, for example, where a pre-drilled hole is available and used for receiving the shaft **1106.**

In one example, the unitary fiducial marker **1100** is made from substantially pure or alloyed titanium, substantially pure or alloyed stainless steel, and/or a ceramic. In one example, the resulting substantially spherical head **1102** is radiolucent and/or radiographically imagable and viewable using computed tomography (CT).

In the example of FIG. **11****,** the unitary fiducial marker **1100** includes an imagable locator head **1102** that is spherical (or otherwise shaped) for obtaining accurate location information (e.g., of its center). The head **1102** also includes a receptacle (such as the divot **1104**) that is shaped for receiving a mating portion (e.g., tip **302**) of a positioning instrument (e.g., wand **300**) during patient registration. Therefore, the unitary fiducial marker **1100** (with integrated imaging and registration divot) in the example of FIG. **11** avoids having to replace an imagable portion of a two-piece fiducial marker (used during preoperative imaging) with a separate registration divot (used during patient registration in the operating room). This simplifies an image-guided surgical procedure using the unitary fiducial marker **1100** having both the imagable head **1102** and the integrated divot **1104.** Such simplification should help lower the cost of the image-guided surgical procedure.

FIG. **13** is a perspective view of modified unitary fiducial marker **1100.** In this example, the shaft **1106** includes a threaded distal portion **1300** and an unthreaded proximal portion **1302.** The unthreaded proximal portion **1302** distances the head **1102** from the surface into which the threaded distal portion **1300** is screwed. In this example, the unthreaded proximal portion **1302** of the shaft **1106** is of a larger cylindrical diameter than the tapered threaded distal threaded portion **1300** of the shaft **1106.** This forms a circular shoulder or seat **1304** at the base of the unthreaded proximal portion **1302** where it meets the threaded distal portion **1300.** When the seat **1304** is of a larger diameter than the major diameter of the threads **1110,** the seat **1304** provides a shoulder acting as a depth stop that inhibits the fiducial marker **1100** from being further advanced into the bone, such as by an accidental impact to the head **1102** of the fiducial marker **1100** that produces a mechanical shock.

In one example, several fiducial markers **1100** are packaged and sold together as a kit. In one such example, such a kit includes two or more different fiducial markers **1100** having different lengths of the unthreaded proximal portion **1302** of their respective shafts **1106.** This accommodates patients having different skin or scalp thicknesses. For example, it may be desirable to keep the head **1102** portion of the fiducial marker **1100** above the patient's skin or scalp, while remaining as close to the skull as possible. If this is desired, it can be accomplished by selecting from the kit a particular fiducial marker **1100** having an appropriate shaft **1106** length to accommodate the skin or scalp thickness of the patient.

In this example, the seat **1304** includes a circular groove, channel, or kerf **1306.** In this example, the kerf **1306** extends along the seat **1304** circumferentially around the threaded distal portion **1300.** The kerf **1306** accommodates therein loose bone fragments that are channeled upward by the threads **1110** when the fiducial marker **1100** is being screwed into the skull. Such groove, channel, or kerf **1306** for accommodating channeled bone fragments could similarly be incorporated into a distal side of the head **1102** in the examples of FIGS. **10 -11****,** in which the threaded portion of the shaft **1106** extends directly from the head **1102.**

### Example 5

FIG. **14** is a perspective view of an optional imagable plug **1400.** FIG. **15** is a side view of the optional imagable plug **1400,** which can be made from the same material as the head **1102,** if desired. The imagable plug **1400** is sized and shaped to be inserted into the divot **1104** during imaging such that the head **1102** presents a uniformly shaped imagable sphere to the imaging modality. This assists in easier location of the centroid of the spherical combination of the head **1102** and the plug **1400,** but is not believed to be required. In this example, the imagable plug **1400** is then removed during registration, thereby permitting access to the divot **1104.** In one example, the plug **1400** includes fins **1402** that are sized and shaped for engaging the corresponding slots **1108.** In an alternative example, however, the fins **1402** are omitted.

In an alternative example, the imagable plug **1400** is made from a material having a slightly or substantially different imaging contrast property from the material comprising the rest of the head **1102.** In this manner, an image of the fiducial marker can be obtained in which the divot **1104** appears with a different imaging contrast than the rest of the head **1102.** This shows the user where the divot **1104** is located within the image.

### Example 6

FIG. **16** is a perspective view of an optional hydrophilic or hygroscopic foam or other magnetic resonance (MR) imagable cover **1600** for slipping over the substantially spherical head **1102.** In this example, the fluid/gel-carrying, fluid/gel-absorbing, or other fluid/gel-incorporating cover includes a circular or similar opening **1602** permitting the shaft **1106** to extend therethrough. In one example, a sterile and biologically safe magnetic resonance (MR) imagable fluid/gel is soaked into the cover **1600** either before or after it is slipped over the head **1102.** This allows the head **1102** to be imaged by MR as well as CT. In an alternative example, such multi-modality of imaging is similarly implemented using a preformed MR-imagable or other coating upon the head **1102,** thereby avoiding any need for slipping a separate cover **1600** over the head **1102.** Such a fluid/gel-carrying, fluid/gel-absorbing, fluid/gel-incorporating, or other MR-imagable or other coating could be formed on the external spherical portion of the head **1102,** or could additionally be formed in the divot **1104** as well. Examples of suitable coatings capable of soaking up an MR-imagable fluid or gel include, by way of example, but not by way of limitation: foam, silicone, etc. Examples of MR imagable fluids for soaking into the cover **1600** (or coating) include, by way of example, but not by way of limitation: sterile saline, sterile saline or another fluid or gel mixed with gadolinium or another MR-imaging enhancing substance, etc.

### Example 7

FIGS. **17 -18** are side cross-sectional and top views, respectively, of an alternative example of a fiducial marker **1700** that is similar in certain respects to the example of FIG. **13****.** In FIGS. **17 -18****,** the fiducial marker **1700** includes a substantially spherical head **1702.** The head **1702** includes a conical or other divot **1704** at its proximal side, and a shaft **1706** extending outwardly from its distal side. In this example, the shaft **1706** includes a proximal portion **1708** and a threaded distal tip portion **1710.** The proximal portion **1708** and the threaded distal tip portion **1710** are separated by a shoulder or other seat **1712,** such as described above. In this example, the divot **1704** of the head **1702** includes rotational engagement features, such as slots **1714,** for receiving a Phillips and/or flathead screwdriver or other driver. Alternatively, an Allen-type receptacle, or any other rotational engagement feature could be used for receiving another driver.

In the example of **FIGS.17 -18****,** the head **1702** is made of a different material than the shaft **1706.** In one example, the different materials are selected to provide different image contrasts on a particular imaging modality (e.g., an MR image, a CT image, or even both types of images). In one such example, the head **1702** is relatively more highly visible on the particular imaging modality, and the shaft **1706** is less highly visible on the particular imaging modality.

In one example, this is effected by using a titanium shaft **1706** that includes a proximally projecting post **1716.** In one example, a proximal end of the post **1716** provides the slots **1714,** as illustrated in FIG. **17****.** In another example, the slots are instead incorporated into the head **1702.** In this example, the head **1702** is a plastic sphere-like object that is insert-molded or otherwise formed about the post **1716.** In one example, the external surface of the post **1716** is knurled or roughened to promote adhesion of the head **1702** to the post **1716,** such as during the insert-molding process. In one example, the head **1702** is highly MR-visible, while the shaft **1706** is not so highly MR-visible, but instead is radiolucent. In addition to insert-molding, other techniques for affixing the head **1702** to the shaft **1706** include, without limitation, gluing, casting, spin-welding, and ultrasonic welding. In yet another example, the post **1716** is threaded, and the head **1702** is threaded and glued onto the post **1716.**

### Example 8

FIGS. **19 - 23** illustrate various distal tip configurations and techniques of attaching fiducial markers to bone. FIG. **19** illustrates a side view of a fiducial marker **1900** that includes a self-drilling and self-tapping threaded distal tip portion **1902.** This example may additionally include a vertical flute-like cutout, as discussed above, for enhancing its self-drilling capability. The head **1903** of the fiducial marker **1902** includes a conical or other divot **1904** and associated slots **1906** or other rotational engagement features for driving the fiducial marker **1900** into bone.

FIG. **20** illustrates a side view of a fiducial marker **2000** that includes a threaded distal tip portion **2002** that need not be self-tapping and/or self-drilling, such as for use when a hole as been pre-drilled into bone for receiving the tip portion **2002.** In one such example, the distal tip portion **2002** is neither self-tapping, nor self-drilling. In another such example, the distal tip portion **2002** is self-tapping, but is not self-drilling.

FIG. **21** illustrates a side view of a fiducial marker **2100** that includes a barbed or other distal tip portion **2102** enabling the fiducial marker **2100** to be driven into bone like a nail or a staple-that is, without needing any rotation. In one example, barbs **2104** help retain the distal tip portion **2102** within the bone. In another example, a nail-like distal tip portion **2102** is used instead. The nail-like distal tip portion **2102** may include a faceted point. In another example, the nail-like distal tip portion **2102** includes anti-rotation features that do not substantially inhibit the distal tip portion **2102** from being driven into bone, but which inhibit rotation after the distal tip portion **2102** has been driven into bone. The fiducial marker **2100** may be removed by grasping and pulling the proximal head **1903,** such as with a staple-puller-like tool. Therefore, this example need not include the slots **1906** or other rotational engagement features because rotation is not needed for inserting or removing the fiducial marker **2100.**

FIG. **22** illustrates a side view of a fiducial marker **2200** having a distal tip portion **2202** that includes tangs **2204A-B,** or another laterally expandable retention element. In one example, the tangs **2204A-B** are pushed outward by an ascending and/or descending longitudinally extending internal rod **2206** that pushes upward or downward against tapered internal shoulders of each of the tangs **2204A-B.** This, in turn, pushes the tangs **2204A-B** laterally outward in opposite directions. The rod **2206** extends longitudinally through an interior passage **2208** of a shaft **2210.** The shaft **2210** extends between the distal tip **2202** and a head **2212** portion of the fiducial marker **2200.** In one example, the rod **2206** terminates at a proximal externally threaded drive head **2214** that engages an internally threaded portion of the head **2212.** The drive head **2214** includes screwdriver slots or one or more other rotational engagement features for turning the drive head **2214.** In one example, turning the drive head **2214** in a clockwise direction moves the drive head **2214** closer to the distal tip **2202** of the fiducial marker **2200.** This pushes the rod **2206** downward, which, in turn, pushes the tangs **2204A-B** outward to grip bone surrounding a pre-drilled hole into which the distal tip **2202** has been inserted. In another example, turning the drive head **2214** in a counter-clockwise direction moves the drive head **2214** away from the distal tip **2202** of the fiducial marker **2200.** This pulls the rod **2206** upward, which, in turn, pushes the tangs **2204A-B** outward to grip bone surrounding a pre-drilled hole into which the distal tip **2202** has been inserted.

FIG. **23** illustrates a side view of a fiducial marker **2300,** similar to the fiducial marker **2200** of FIG. **22****,** but having a self-tapping and/or self-drilling externally threaded distal tip portion **2302,** such as for being introduced into bone without using a pre-drilled hole. The head **2212** of the fiducial marker **2300** of FIG. **23** also includes slots **1906** or other rotational engagement features for rotationally driving the fiducial marker **2300** into bone, such as by using a screwdriver. Then, the tangs **2204A-B** are forced outward as described above with respect to the fiducial marker **2200** of FIG.

### Example 9

After a fiducial marker has been introduced into a patient's skull or other bone, it may be desirable to protect the fiducial marker, such as against accidental shocks or impacts, "twiddling" by the patient, etc.

FIG. **24** is a side cross-sectional view of a fiducial marker **2400** having a substantially spherical head **2402** that includes an internally threaded proximal divot **2406,** and a shaft **2408** extending outward from a distal side of the head **2402** toward a distal tip **2410** that has been threaded into a portion of the subject's skull **2412.** In this example, a protective cap **2414** has been threaded into the divot **2406.** The protective cap **2414** includes a disk-like top portion **2416** and a cylindrical circumferential skirt **2418.**

FIG. **25** is a side cross-sectional view of a further example of the protective cap **2414** in which the skirt **2418** includes an adjustable height outer cylindrical circumferential skirt **2420.** In this further example, threads on the internal portion of the skirt **2418** engage threads on the outer portion of the skirt **2420,** providing height adjustability to accommodate different scalp thicknesses. In use, the fiducial marker **2400** is first affixed to the subject's skull, then the protective cap is threaded into the divot **2406,** and then the outer skirt **2420** is lowered to the appropriate height for the particular patient's scalp thickness.

FIG. **26** is a side cross-sectional view of another example of a protective cap **2600,** which is disposed about a fiducial marker **2602** that has been affixed to a subject's skull. In this example, the cap **2600** includes a proximal disk portion **2604,** a cylindrical circumferential portion **2606,** and a distal base ring flange portion **2608.** The distal base ring flange portion **2608** includes a self adhesive coating **2610** on its distal side. This allows attachment of the protective cap **2600** to the patient's scalp.

FIG. **27** is a perspective view of a protective collar **2700** that can be disposed about a fiducial marker **2702** that has been affixed to a subject's skull. In this example, the protective collar **2700** includes a disk-like base **2704** and a circumferential cylindrical sidewall **2706** rising upward from a perimeter of the base **2704.** The collar **2700** includes a radial slot **2708** in the base **2704.** A first end of the radial slot **2708** terminates at an orifice **2710** at the center of the base **2704.** A second end of the radial slot **2708** terminates at a peripheral slot **2712,** at substantially a right angle thereto, extending up the sidewall **2706** of the collar **2700.** The collar **2700** is somewhat flexible (e.g., made of plastic), and the peripheral slot **2712** and the radial slot **2708** are sized and shaped to pass the shaft **2714** of the fiducial marker **2702** through to the center orifice **2710,** where it is seated. When the shaft **2714** is seated within the center orifice **2710,** a height **2716** of the sidewall **2706** of the collar **2700** is greater than a height **2718** between a top of the fiducial marker **2702** and the patient's scalp **2720.** When the collar **2700** has been disposed about the fiducial marker **2702,** it protects the fiducial marker **2702** against a mechanical impact.

FIG. **28** is a perspective view of the collar **2700** further including a disk-like cap **2800 that** fits snugly over and around the top of the collar **2700** to house and substantially enclose the fiducial marker **2702** disposed within the collar **2700.** The cap **2800** is not required, but it provides additional structural strength and helps keep clean the incision through which the fiducial marker **2702** was introduced.

FIG. **29** is a perspective view of an alternate example of a collar **2900,** similar to that illustrated in **FIGS. 27 - 28****,** but that omits the radial slot **2708** and the peripheral slot **2712.** In this example, the protective collar **2900** includes a disk-like base **2904** and a circumferential cylindrical sidewall **2906** rising upward from a perimeter of the base **2904.** The collar **2900** includes an orifice **2910** at the center of the base **2904.** The flexible base **2904** includes small incisions **2920** extending radially from the orifice **2910** to permit the head **2922** portion of the fiducial marker **2702** (which is larger than the orifice **2910)** to pass through the orifice **2910.** The orifice **2910** is sized to accommodate the shaft **2714** portion of the fiducial marker **2702** snugly therein. When the collar **2900** is seated against the scalp **2720,** a height **2916** of the sidewall **2906** of the collar **2900** is greater than a height **2918** between a top of the fiducial marker **2702** and the scalp **2720** of the patient. When the collar **2900** has been disposed about the fiducial marker **2702,** it protects the fiducial marker **2702** against a mechanical impact, etc. The collar **2900** can also be used in conjunction with the cap **2800** illustrated in FIG. **28****,** as discussed above.

FIG. **30** is a perspective view illustrating an example of a headband **3000** for protecting fiducial markers from mechanical impact. The headband **3000** is sized and shaped to fit around the skull of a subject **3002.** The headband includes one or more fixation straps **3003,** e.g., using Velcro to attach opposing sides of the headband **3000.** In one example, the headband **3000** includes one or more pre-formed holes **3004,** which are located in relationship to each other in a manner to be suitable for placing image-guided surgical (IGS) fiducial markers at the locations **3006** of the holes when the headband **3000** is placed about the subject's head. In an alternative example, the headband **3000** does not include such holes **3004.** Instead, the user cuts holes in the headband **3000** as desired for locating the fiducial markers. In yet another example, the holes **3004** are replaced by perforation openings, so that the underlying fiducial marker only pokes through the headband as much as is needed.

FIG. **31** is a side view illustrating an example of a tubular protective guide collar **3100.** The guide collar **3100** carries a fiducial marker **3102.** The guide collar **3100** is useful for holding and guiding the fiducial marker **3102** while it is being affixed to the patient's skull, as well as for protecting the fiducial marker **3102** after it has been affixed to the patient's skull. In this example, the tubular guide collar **3100** includes an inner diameter **3104** that is large enough to receive the head **3106** of the fiducial marker **3102.** An intermediate portion of the guide collar **3100** includes a circumferential neck **3107.** The neck **3107** has a slightly smaller inner diameter than the diameter of the head **3106.** However, the neck **3107** is flexible, deformable, and/or compliant enough to pass the head **3106** through the neck **3107** when the fiducial marker **3102** is affixed to the patient's skull-without pulling the fiducial marker **3102** loose from the patient's skull. This can be accomplished by constructing the guide collar **3100** of a somewhat compliant plastic, and providing appropriate neck dimensions for a particular fiducial marker head **3106.** The guide collar **3100** also optionally includes a distal flange **3108,** such as to provide additional stability and to enhance vertical orientation of the guide collar **3100.** The user can hold the guide collar **3100** in place, such as by pressing two fingers against the flange **3108** to hold it against the patient's scalp. This properly holds straight and orients the fiducial marker **3102** as it is threaded into or otherwise affixed to the subject's skull. It promotes an orthogonal orientation of the fiducial marker **3102** with respect to the subject's skull.

FIG. **32** is a flow chart illustrating one example of using the guide collar **3100.** At **3200,** the fiducial marker **3102** is dropped into a proximal end of the guide collar **3100.** The fiducial marker **3102** falls through the proximal tubular portion and comes to rest against the interior portion of the neck **3106,** as illustrated in FIG. **31****.** Then, at **3202,** a distal end of the guide collar **3100** is positioned against the subject's scalp, such as by pressing down against the optional flange **3108.** At **3204,** the fiducial marker **3102** is affixed to the subject's skull, such as by inserting a screwdriver tip into the proximal end of the guide collar **3100** and into corresponding screwdriver slot(s) in the head **3106** of the fiducial marker **3102,** and screwing the fiducial marker **3102** into the patient's skull. At **3206,** the guide collar **3100** can be left in place, if desired, to protect the fiducial marker **3102** against a mechanical impact. When the fiducial marker **3102** is affixed to the patient's skull, and the flange **3108** rests against the patient's scalp, the height **3110** of the guide collar **3100** is greater than the corresponding height of the fiducial marker **3102,** such that the fiducial marker head **3106** is still located within the tubular guide collar **3100.** This protects the fiducial marker **3100,** such as from an axial mechanical impact that otherwise might potentially drive the fiducial marker **3100** deeper into the patient's skull. At **3208,** the guide collar **3100** can be removed while leaving the fiducial marker **3102** affixed to the subject's skull. This can be accomplished by grasping and pulling on the guide collar **3100,** or by prying under the flange **3108.** As discussed above, the neck **3107** is sufficiently compliant to pass the head **3106** from the proximal portion of the hourglass-shaped guide collar **3100** to its distal portion. This allows the guide collar **3100** to be removed over the top of the fiducial marker **3102** while leaving it in place. Alternatively, the fiducial marker **3102** could be affixed to the subject without using the guide collar **3100,** and the guide collar **3100** could later be snapped into place over the fiducial marker **3102** to protect it against a mechanical impact, as discussed above.

FIG. **33** is a perspective view of an alternative guide collar **3300.** In this example, the guide collar **3300** has more than one piece. In FIG. **33****,** the guide collar **3300** includes a cylindrical tubular guide base **3302** and a cylindrical height extender **3304.** In this example, the cylindrical tubular guide base **3302** includes an optional distal flange **3306.** The guide base **3302** includes a side access slot **3308** that is sized and shaped to pass a shaft portion **3112** of the fiducial marker **3102.** The flange **3310** includes a similar slot **3310,** which is aligned with the slot **3308.** The cylindrical height extender **3303** can be press-fit over the guide base **3302** snugly enough to hold these two pieces together until they are again pulled apart by the user.

FIG. **34** is a side sectional view of the guide base **3302,** the height extender **3304,** a fiducial marker **3102,** and a screwdriver shaft **3400.** FIG. **35** is a flow chart illustrating one example of using the guide base **3302** and the height extender **3304** of FIGS. **33 - 34****.** At **3500,** the fiducial marker **3102** is inserted into the guide base **3302,** either by dropping it in the top or by inserting its shaft laterally through the side access slot **3308.** At **3502_{.}** a distal portion of the guide base **3302** is placed against the subject's scalp and held in place, such as by pressing down against the optional flange **3306.** At **3504,** the fiducial marker **3102** is affixed to the subject's skull, such as by screwing it in such as illustrated in FIG. **34****.** At **3506,** the height extender **3304** is slid over and snugly press-fitted around the guide base **3302.** As illustrated in FIG. **34****,** the height extender **3304** is taller than the affixed fiducial marker **3102.** In this manner, the height extender **3304** protects the fiducial marker **3102** against a mechanical impact, such as an axial blow that might otherwise drive the fiducial marker **3102** deeper into the patient's skull. At **3508,** the height extender **3304** is removed by axial pulling. At **3510,** the guide base **3304** is laterally removed, thereby passing the shaft of the fiducial marker **3102** out of the slot **3308.**

FIG. **36** is a schematic illustration of a subject **3600** with one or more fiducial markers **3602** affixed to the subject's skull. As discussed above, in one example, the fiducial markers **3602** include substantially spherical heads with integrated conical divot receptacles therein for mating to a remotely detectable positioning instrument. FIG. **37** illustrates schematically one example of how such fiducial marker head images **3700** appear on an image created by MR, CT, or another imaging modality. For registering the patient, it is useful to know the center locations of the fiducial marker head images **3700.** However, the presence of the integrated divot may confound the fiducial marker head images **3700** somewhat.

FIG. 33 illustrates schematically one example of a template **3800** including one or more concentric rings with a center indicator (such as a bull's-eye pattern or the like) such as for assisting the user in locating the center of the fiducial marker head images **3700.** In one example, the template **3800** is implemented on a physical media (e.g., a transparency) that is placed over the fiducial marker head image **3700** (e.g., on a computer display, such as the IGS workstation **400**). In another example, the template **3800** is implemented by computer software (e.g., as a mouse-draggable icon or feature on a computer display, such as the IGS workstation **400**) that is moved using a mouse or other computer input device to place it over a fiducial marker head image **3700.** In either example, the template **3800** is concentrically aligned (e.g., using one or more of its concentric rings or similar curves for aligning with a two-dimensional image of a sphere) to one of the fiducial marker head images **3700.** This provides an indication of the center of that fiducial marker head image 3700. In the physical media example, the user moves a cursor to align the fiducial marker head image 3700 with the center of the template, and clicks a mouse button to select the center of the fiducial marker head image 3700. In the software template 3800 example, the user clicks a mouse button when the software template 3800 is aligned with a center of the fiducial marker head image 3700 to select the same. The selected center of the fiducial marker head image 3700 is then used, during the patient registration process, to correlate to the physical location of the apex of the conical divot, as located by the tip of the positioning device that mates thereto, as discussed above.

Although the above examples illustrated with respect to FIGS. 11-38 have been discussed with particular emphasis on a spherical imagable fiducial marker with integrated receptacle and bone screw, it should be understood that alternative examples are implemented using a cylindrical or faceted columnar shaped fiducial marker with integrated receptacle and bone screw. Moreover, in a further example, such fiducial markers include reflective outer surfaces that are recognizable by a remote positioning system, as discussed elsewhere in this document. Still further, such fiducial markers can incorporate anti-microbial properties, such as by using an anti-microbial coating, or using silver or silver-based alloys for their manufacture.

FIG. 39 is a schematic diagram illustrating generally one example of a fiducial marker 3900 and a positioning system 3902. In this example, the fiducial marker 3900 includes an assembly comprising a mounting base 3904 and a locator 3906. The mounting base 3904 includes a self-tapping or other externally threaded distal portion 3908. This permits the base 3904 to be screwed into a patients skull or another desired surface. A proximal portion 3990 includes a male or female receptacle 3912. The receptacle 3912 is sized and shaped to receive a complementary male or female receptacle 3914 located on a distal portion of the locator 3906. In the example of FIG. 39, the receptacle 3912 is an internally threaded or other orifice, and the receptacle 3914 is an externally threaded or other prong,

In the example of FIG. 39, the locator 3906 includes a shaft 3916 between the distal receptacles 114 and a proximal imageable sphere 3918. The imageable sphere 3918 is made from, or contains, a material that provides good contrast on one or more imaging modalities. Examples of suitable imaging systems include, by way of example, but not by way of limitation, magnetic resonance (MR) imaging systems, computed tomography (CT), positron emission tomography (PET), and single photon emission computed tomography (SPECT), X-ray, fluoroscopy, or other radiographic imaging systems, ultrasonic imaging systems, and the like. These imaging modalities permit acquisition of an image of a volume of interest, such as a portion of a subject's brain. The acquired image includes a visible image of the imageable sphere **3918,** providing a landmark that is located on the subject's skull.

In the example of FIG. **39****,** the positioning system **3902** includes a positioning instrument, which is also sometimes referred to as a positioning wand **3920.** The wand **3920** includes a distal cap **3922.** The distal cap **3922** includes a substantially semispherical orifice **3924.** The orifice **3924** is sized and shaped to fit snugly over the imageable sphere **3918** such that a reference point **3926** aligns with a center **3928** of the imageable sphere **3918.** The wand **3920** includes a shaft **3930** between the distal cap **3922** and a proximal end **3932.** The proximal end **3932** of the wand **3920** includes positioning locators **3934A-C** that are remotely detectable by a detector portion of the positioning system **3902.** In this example, the positioning system **3902** is implemented as an optical positioning system and the detector is implemented as a camera **3936.** The positioning locators **3934A-C** are spherical or other reflectors (or, alternatively, an energy source, such as light-emitting diodes (LEDs)) that are illuminated by a light source **3938** for detection by the camera **3936.** The camera **3936** feeds information about the location of the positioning locators **3934A-C** to an image-guided surgical (IGS) computer workstation **3938.**

The positioning locators **3934A-C** are located in a predetermined fixed arrangement with respect to each other and with respect to the reference point **3926.** Therefore, recognizing the locations of the positioning locators **3934A-C** using the positioning system **3902** allows computation of the location of the reference point **3926.** Therefore, when the cap **3922** is placed upon the sphere **3918,** this, in turn, permits computation of the location of the center point **3928** of the sphere **3918.** FIG. **39** illustrates the positioning locators **3934A-C** in a very general conceptual way. One or more of the positioning locators will typically be individually mounted on one or more respective arms extending radially or otherwise from the proximal portion **3932** of the wand **3920,** such as illustrated in FIG. **43****.**

FIG. **40** is a flow chart illustrating generally one example of using devices such as are shown in the example of FIG. **39****.** In the example of FIG. **40**, at **4000,** several bases (typically at least three or four) are screwed into the subject's skull or other desired surface, such as by using a socket that engages an externally faceted surface such as a hex head of the proximal portion **3910** of the base **3904.** At **4002,** a locator **3906** is attached to each one of the bases **3904.** At **4004,** at least one imaging modality is used to take one or more preoperative or other images of the subject's skull, or other desired volume of interest. Images of the locators **3906** typically appear with good contrast on the images of the volume of interest. This image information is feed to the IGS workstation **3938** for computing the locations, in the three-dimensional space of the images, of the centers **3928** of the spheres **3918.** The subject is then moved to the operating room. At **4006,** the cap **3922** of the wand **3920** is placed over each of the spheres **3918** to obtain the locations of their centers **3928** to register the three dimensional space in which the patient is located to the three dimensional space of the preoperative images. This allows the preoperative images to be used for stereotactically guiding surgical operations on the subject in the operating room. Among other things, the devices shown in FIG. **39** avoid any need for replacing the locators **3906** with a golf-tee-like "divot" or "localization cap" for receiving the wand **3920.** This, in turn, reduces the complexity and cost of the stereotactic procedure.

FIG. **41** is a schematic diagram illustrating generally an alternative example of a positioning wand **4100,** with the base **3904** and the locator **3906.** The positioning wand **4100** includes a ball **4102** and socket **4104** or other joint. The ball **4102** and socket **4104** pivot about a center reference point **4106.** In this example, the ball **4102** is the same size and shape as the sphere **3918** of the locator **3906.** Distal to the ball **4102** is a shaft **4108** that is the same size as the shaft **3916** of the locator **3906.** Distal to the shaft **3916** is a prong (or other male or female receptacle) **4110** that is the same length as the prong-like male receptacle **3914** of the locator **3906.** In this example, unlike the externally threaded prong-like receptacle **3914** of the locator **3906,** the prong **4110** is not threaded. This permits the prong **4110** to be easily inserted into and removed from the receptacle **3912** of the base **3904.**

FIG. **42** is a flow chart illustrating generally one example of using the devices illustrated in FIG. **41** and FIG. **39****.** At **4200,** the bases **3904** are screwed in, such as discussed above. At **4202,** the locators 3906 are attached to respective bases **3904,** such as discussed above. At **4204,** the subject is imaged together with the locators **3906,** such as discussed above. The subject is then moved into the operating room, such as discussed above. At **4206,** the locators **3906** are unscrewed or otherwise removed from the respective bases **3904.** At **4208,** the subject is registered to the images. This includes inserting the **tip'4110** of the positioning wand **4100** into the receptacle **3912** of each of the respective bases **3904.** The positioning locators **3934** on the wand **4100** are in a known relationship to the pivoting center reference point **4106,** which, in turn, occupies the same location as the center **3928** of the sphere **3918** when the locator **3918** was inserted within the base **3904.** In this manner, by using the positioning system **3902** to determine the locations of the positioning locators **3934** on the wand **4100,** the center point **3928** that was occupied by each of the locators **3906** can be computed by the IGS workstation **3938.** Again, among other things, this process avoids any need for replacing the locators **3906** with a golf-tee-like "divot" or "localization cap" for receiving the wand **4100.** This, in turn, reduces the complexity and cost of the stereotactic procedure.

FIG. **43** is a schematic diagram illustrating generally an alternative example of a base **4300,** a locator **4302,** and a positioning wand **4304.** The base **4300** is similar, in certain respects, to the base **3904.** However, in this example, the base **4300** includes a receptacle **4306** that includes a distal conical "divot" **4308,** such as for receiving a pointed distal tip **4310** of the positioning wand **4304.** The locator **4302** includes two imageable spheres **4312A-B.** The imageable spheres **4312A-B** are respectively located on middle and proximal portions of a shaft **4313.** The spheres **4312A-B** include respective centers **4314A-B** that define a line therethrough. When a distal tip **4315** of the locator **4302** is threaded or otherwise inserted into the receptacle **4306** of the base **4300,** the line through the centers **4314A-B** extends through the apex (point of maximum depression) of the conical divot **4308.** In FIG. **43****,** the positioning wand **4304** includes a shaft **4318** extending proximally from the distal tip **4310** and terminating at or near radial arms **4320A-C.** The radial arms **4320A-C** carry respective positioning locators **4322A-C.**

The method described with respect to FIG. **42** can also be used with the devices shown in FIG. **43****.** The images of the subject (or other volume of interest) with the locators **4302** permit computation of each of the centers **4314A-B** and of the line defined therebetween. The location of the apex of the divot **4308** is located on this line at a known predetermined distance from the centers **4314A-B.** During registration, at **4208,** in which the tip **4310** of the positioning wand is inserted into the divot **4308** of each of the respective bases, the actual locations of the apexes of the divots **4308** is computed, because the tip **4310** of the positioning wand is in a known spatial relationship to the positioning locators **4322.** These points of the apexes of the divots **4308** are registered to corresponding points in the images that are extrapolated along the line defined by the centers **4314** of the imageable spheres **4312A-B.** Again, among other things, this process avoids any need for replacing the locators **3906** with a golf-tee-like "divot" or "localization cap" for receiving the wand **4304.** This, in turn, reduces the complexity and cost of the stereotactic procedure.

FIG. **44** is a schematic diagram illustrating generally an alternative example of another locator **4400** with the base **3904.** In this example, the locator **4400** includes an imageable sphere **4402.** The imageable sphere **4402** includes a removable cone **4404** that forms an imageable portion of the imageable sphere **4402.** Removing the cone **4404** creates a conical orifice (also referred to as a divot) **4406.** The conical orifice **4406** has an apex located at a center **4408** of the imaging sphere **4402.** The conical orifice **4406** is sized and shaped to permit a tip **4310** of a positioning wand **4304** to be received therein for performing registration. In one example, the cone **4404** snap-fits into the conical orifice **4406,** such as by a beveled proximal circumferential rim **4409** that engages a lip **4410** located circumferentially about the proximal base portion of the inverted conical orifice **4406,** as illustrated in FIG. **39****.** In one example, the imageable cone **4404** and/or the imageable sphere **4402** includes a small orifice **4500,** such as illustrated in the top view of FIG. **45****,** to facilitate prying the imageable cone **4404** out of the imageable sphere **4402,** such as by using a pick or like instrument to perform this removal.

FIG. **46** is a schematic diagram illustrating generally an alternative example of a locator **4600** including an imageable sphere **4602** with a removable imageable cone **4604.** In this example, a proximal portion of the cone **4604** includes external threads **4606** for engaging internal threads **4608** of a conical orifice **4610** providing a divot for receiving a tip **4310** of a positioning wand **504.** An apex of the conical orifice **4610** corresponds to the center **4612** of the imageable sphere **4602.** FIG. **47** illustrates a top view of the cone **4604,** including an orifice **4700** for receiving a pick or other instrument for unscrewing the cone **4604** from the sphere **4602** for removing it therefrom.

FIG. **48** is a flow chart illustrating generally one example of a method for using the devices illustrated in FIGS. **44-47****.** In FIG. **48**, at **4800,** the bases **3904** are screwed in, such as discussed above. At **4802,** the locators **4400** or **4600** are attached to respective bases **3904,** such as discussed above. At **4804,** the subject is imaged together with the locators **4400** or **4600,** such as discussed above. The subject is then moved into the operating room, such as discussed above. At **4806,** the imageable cones **4404** or **4604** are pried, unscrewed, or otherwise removed from the respective bases **3904.** At **4308,** the subject is registered to the images. In one example, this includes inserting the tip **4310** of the positioning wand **4304** into the orifice **4410** or **4610,** such that the tip **4310** is located at the center of the imageable sphere **4402** or **4602.** The positioning locators **4322** on the wand **4304** are in a known relationship to the tip **4310** located at the center **4408** or **4612** of the imageable sphere **4402** or **4602.** In this manner, by using the positioning system **3902** to determine the locations of the positioning locators **4322** on the wand **4304,** the center point **4408** or **4612** can be computed by the IGS workstation **3938.** Again, among other things, this process avoids any need for replacing the locators **4400** or **4600** with a golf-tee-like "divot" or "localization cap" for receiving the wand **4304.** This, in turn, reduces the complexity and cost of the stereotactic procedure. Moreover, accuracy may be enhanced because the tip **4310** is located at the actual center **4408** or **4612** of the imageable sphere **4402** or **4602,** rather than using an intermediate element such as a golf-tee-like "divot" or "localization cap" for receiving the wand **4304.**

FIGS. **49A - 49D** are schematic diagrams illustrating generally other examples of locators **4900A-D** having imageable spheres **4902A-D** that include removable imageable components that allow direct access to the centers **4904A-D** of the respective imageable spheres **4902A-D,** such as for registration by touching a wand tip **4310** thereto. FIG. **49A** shows a removable imageable sphere **4906A** that is snap-fitted to an imageable inverted cone **4908A** on a proximal portion of the shaft **3914.** FIG. **49B** shows a removable imageable sphere **4906B** that is threaded onto an imageable inverted cone **4910B** on a proximal portion of the shaft **3914.** The apexes of the inverted cones **4908A** and **4908B** respectively define the centers **4904A** and **4904B** of the imageable spheres **4902A** and **4902B.** FIG. **49C** shows a removable imageable hemisphere **4910** that is snap-fitted to a complementary imageable hemisphere **4912** that is attached to a proximal portion of the shaft **3914.** The snap-fitting provides a small male or female receptacle at the center of the imageable sphere **4902C** to which a wand tip can be touched during registration. FIG. **49D** shows a removable imageable sphere **4914** that is snap-fitted to an imageable post **4916** extending from a proximal end of the shaft **3914.** The snap-fitting provides a small male or female receptacle at the center of the imageable sphere **4902C** to which a wand tip can be touched during registration. The devices shown in FIGS. **49A-C** can be used with the method analogous to that described with respect to the flow chart of FIG. **48****.**

FIG. **50** is a schematic diagram illustrating generally an example of a fiducial marker assembly **5000.** In this example, the fiducial marker assembly **5000** comprises a mounting base **5002,** which is attached to a skull **5004,** and an imageable fiducial marker locator **5006.** The locator **5006** includes an imageable sphere **5008.** A removable imageable cone **5010** portion of the imageable sphere **5008** permits access to the center **5012** of the imageable sphere **5008,** such as during registration.

In the example of FIG. **50****,** the cone **5010** is threaded into the other portions of the sphere **5008.** The cone **5010** is attached to a protective cap **5014.** In the example of FIG. **50****,** the cap **5014** includes a proximal disk **5016,** tangentially extending radially from the removable imageable cone **15010** portion of the imageable sphere **5008.** A sleeve **5018** extends from the circumference of the disk **5016** toward the skull **5004.** The cap **5014** protects portions of the fiducial marker assembly **5000** from damage. The cap **5014** is either made of a material that is imageable (like the cone **5010** and the other portions of the sphere 5008) or of a different material that is not imageable, i.e., does not provide good contrast on an imaging modality. In a further example, the sleeve 5018 includes external threads that engage internal threads of a cylindrical skirt 5020, which allows the protective cap 5014 to accommodate different scalp thicknesses.

Although the above examples of positioning were illustrated in conjunction with optical positioning systems, certain aspects of such positioning wands can also be used with a wide variety of other remotely detectable positioning systems, such as electric or magnetic field type positioning systems using electric or magnetic positioning locators, articulated arm type positioning systems, etc.

In further examples, the various above-described locators (e.g., on the subject's skull, or on a wand, as illustrated in Figure 3) alternatively or additionally include an electromagnetic (EM) coil that permits determination of the position of the locator using an EM coil detecting positioning system coupled to an IGS workstation rather than the optical positioning system 412 discussed above.

Moreover, the above techniques are not limited to diagnosis of the human body; the fiducial markers can be used to register the position of any object to a , previously-acquired image of that object.

It is to be understood that the above description is intended to be illustrative, and not restrictive. For example, the above-described embodiments may be used in combination with each other, Many other embodiments will be apparent to those of skill in the art upon reviewing the above description. The scope of the invention should, therefore, be determined with reference to the appended claims,

In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An apparatus comprising:
an imageable fiducial marker (4400; 4600; 5006) including an imageable sphere (4402; 4602; 5008) having a receptacle (4406; 4610);
a base (3904; 5002), the base comprising proximal and distal ends, the distal end including external threads (3908), the proximal end attached to said imageable fiducial marker (4400; 4600; 5006), and
a removable imageable fiducial marker portion (4404; 4604; 5010);
wherein the receptacle (4406; 4610) of the imageable fiducial marker is sized and shaped to receive the removable imageable fiducial marker portion (4404; 4604) and also is sized and shaped to receive a positioning instrument (4304) at the centre of the imageable fiducial marker.

2. The apparatus of claim 1, further comprising a positioning instrument (4304), the positioning instrument comprising a pointed tip (4310) sized and shaped to be received by the receptacle (4406; 4610).

3. The apparatus of claim 2, wherein the positioning instrument (4304) further comprises:
a shaft extending proximally from the pointed tip (4310); and
a plurality of radial arms (4320A-C) extending from the shaft and carrying respective positioning locators (4322,A-C).

4. The apparatus of claim 1, wherein the imageable fiducial marker (4400; 4600; 5006) includes a shaft having a male receptacle that is received within a corresponding female receptacle of said base (3904; 5002), said shaft extending outward from the imageable sphere (4402; 4602; 5008).

5. The apparatus of claim 1, wherein said receptacle (4406; 4610) comprises a substantially conical divot including an apex that is integrally located, with respect to the imageable sphere (4402; 4602; 5008), such that a center of an image of the imagable fiducial marker (4400; 4600; 5006) substantially coincides with the apex of the divot.

6. The apparatus of claim 1, further including a protective cap (5014) sized and shaped for protecting the fiducial marker.

7. The apparatus of claim 6, in which the protective cap (5014) engages the head.

8. The apparatus of claim 6, in which the protective cap (5014) includes a skirt (5020) configured for scalp adhesion.

9. The apparatus of claim 6, in which the protective cap (5014) fits about the shaft.

10. The apparatus of claim 1, wherein said receptacle (4406; 4610) comprises:
a threaded proximal portion (4608); and
a conical distal portion.

11. The apparatus of claim 1, wherein said removable imageable fiducial marker portion (4404; 4604) comprises a cone.

12. A system, comprising:
the apparatus of claim 1;
a light source (3938) configured to illuminate positioning locators (4322A-C) of the positioning instrument (4304); and
a detector (3936) configured to detect light reflected from the positioning locators (4322A-C).

13. A method comprising:
attaching (4802) imageable spheres (4402; 4602; 5008) to respective ones of a plurality of bases (3904; 5002) that have been screwed (4800) into at least one surface of a subject;
imaging (4804) a volume including the imageable spheres (4402; 4602; 5002);
removing (4806) respective imageable portions (4404; 4604; 5010) of the imageable spheres to permits access to centers (4408; 4612; 5012) of the imageable spheres; and
registering (4808) a subject to the images, including touching the centers of the imageable spheres with a positioning instrument (4304).

## Patentansprüche

1. Vorrichtung, die Folgendes umfasst:
eine abbildbare Bezugsmarkierung (4400; 4600; 5006), die eine abbildbare Kugel (4402; 4602; 5008) einschließt, die eine Aufnahme (4406; 4610) hat,
eine Basis (3904; 5002), wobei die Basis ein proximales und ein distales Ende umfasst, wobei das distale Ende Außengewindegänge (3908) umfasst, wobei das proximale Ende an der abbildbaren Bezugsmarkierung (4400;4600;5006) befestigt ist, und
einen abnehmbaren abbildbaren Bezugsmarkierwngsabschnitt (4404; 4604; 5010),
wobei die Aufnahme (4406; 4610) der abbildbaren Bezugsmarkierung dafür bemessen und geformt ist, den abnehmbaren abbildbaren Bezugsmarkierungsabschnitt (4404; 4604; 5010) aufzunehmen, und ebenfalls dafür bemessen und geformt ist, ein Positionierungsinstrument (4304) an dem Mittelpunkt der abbildbaren Bezugsmarkierung aufzunehmen.

2. Vorrichtung nach Anspruch 1, die ferner ein Positionierungsinstrument (4304) umfasst, wobei das Positionierungsinstrument eine scharfe Spitze (4310) umfasst, die dafür bemessen und geformt ist, durch die Aufnahme (4406; 4610) aufgenommen zu werden.

3. Vorrichtung nach Anspruch 2, wobei das Positionierungsinstrument (4304) ferner Folgendes umfasst:
einen Schaft, der sich in Proximalrichtung von der scharfen Spitze (4310) aus erstreckt, und
mehrere radiale Arme (4320A-C), die sich von dem Schaft aus erstrecken und jeweilige Positionierungszeiger (4322A-C) tragen.

4. Vorrichtung nach Anspruch 1, wobei die abbildbare Bezugsmarkierung (4400; 4600; 5006) einen Schaft einschließt, der eine Außenaufnahme hat, die innerhalb einer entsprechenden Innenaufnahme der Basis (3904; 5002) aufgenommen wird, wobei sich der Schaft von der abbildbaren Kugel (4402; 4602; 5008) aus nach außen erstreckt.

5. Vorrichtung nach Anspruch 1, wobei die Aufnahme (4406; 4610) eine im Wesentlichen kegelförmige Vertiefung, einschließlich eines Scheitelpunkts, umfasst, die integral, in Bezug auf die abbildbare Kugel (4402; 4602; 5008), derart angeordnet ist, dass ein Mittelpunkt eines Bildes der abbildbaren Bezugsmarkierung (4400; 4600; 5006) im Wesentlichen mit dem Scheitelpunkt der Vertiefung übereinstimmt.

6. Vorrichtung nach Anspruch 1, die ferner eine Schutzkappe (5014) einschließt, die dafür bemessen und geformt ist, die Bezugsmarkierung zu schützen.

7. Vorrichtung nach Anspruch 6, wobei die Schutzkappe (5014) den Kopf in Eingriff nimmt.

8. Vorrichtung nach Anspruch 6, wobei die Schutzkappe (5014) einen Saum (5020) einschließt, der für die Haftung an der Kopfhaut konfiguriert ist.

9. Vorrichtung nach Anspruch 6, wobei die Schutzkappe (5014) um den Schaft passt.

10. Vorrichtung nach Anspruch 1, wobei die Aufnahme (4406; 4610) Folgendes umfasst:
einen mit Gewinde versehenen proximalen Anschnitt (4608) und
einen kegelförmigen distalen Abschnitt.

11. Vorrichtung nach Anspruch 1, wobei der abnehmbare abbildbare Bezugsmarkierungsabschnitt (4404; 4604) einen Kegel umfasst.

12. System, das Folgendes umfasst:
die Vorrichtung nach Anspruch 1,
eine Lichtquelle (3938), die dafür konfiguriert ist, Positionierungszeiger (4322A-C) des Positionierungsinstruments (4304) zu beleuchten, und
einen Detektor (3936), der dafür konfiguriert ist, das von den Positionierungszeigern (4322A-C) reflektierte Licht zu erfassen.

13. Verfahren, das Folgendes umfaßt:
das Befestigen (4802) von abbildbaren Kugeln (4402; 4602; 5008) an jeweils einer von mehreren Basen (3904; 5002), die in wenigstens eine Fläche eines Subjekts geschraubt (4800) worden sind,
das Abbilden (4804) eines Volumens, das die abbildbaren Kugeln (4402; 4602; 5002) einschließt,
das Abnehmen (4806) von jeweiligen abbildbaren Abschnitten (4404; 4604; 5010) der abbildbaren Kugeln, um einen Zugang zu Mittelpunkten (4408; 4612; 5012) der abbildbaren Kugeln zu gewähren, und
das Ausrichten (4808) eines Subjekts mit den Bildern, einschließlich des Berührens der Mittelpunkte der abbildbaren Kugeln mit Einem Positioniezungsinstrument (4304).

## Revendications

1. Appareil, comprenant :
un repère de cliché imageable (4400 ; 4600 ; 5006), englobant une sphère imageable (4402 ; 4602 ; 5008) comportant un réceptacle (4406 ; 4610) ;
une base (3904 ; 5002), la base comprenant des extrémités proximale et distale, l'extrémité distale englobant des filets externes (3908), l'extrémité proximale étant fixée sur ledit repère de cliché imageable (4400 ; 4600 ; 5006) ; et
une partie de repère de cliché imageable amovible (4404 ; 4604 ; 5010) ;
le réceptacle (4406 ; 4610) du repère de cliché imageable étant dimensionné et formé de sorte à recevoir la partie de repère de cliché imageable amovible (4404 ; 4604 ) et étant en outre dimensionné et formé de sorte à recevoir un instrument de positionnement (4304) au niveau du centre du repère de cliché imageable.

2. Appareil selon la revendication 1, comprenant en outre un instrument de positionnement (4304), l'instrument de positionnement comprenant un embout pointu (4310), dimensionné et formé de sorte à être reçu par le réceptacle (4406 ; 4610).

3. Appareil selon la revendication 2, dans lequel l'instrument de positionnement (4304) comprend en outre :
un arbre, s'étendant dans une direction proximale à partir de l'embout pointu (4310) ; et
plusieurs bras radiaux (4320A-C), s'étendant à partir de l'arbre et supportant des localisateurs de positionnement respectifs (4322A-C).

4. Appareil selon la revendication 1, dans lequel le repère de cliché imageable (4400 ; 4600 ; 5006) englobe un arbre comportant un réceptacle mâle reçu dans un réceptacle femelle de ladite base (3904 ; 5002), ledit arbre s'étendant vers l'extérieur à partir de la sphère imageable (4402 ; 4602 ; 5008).

5. Appareil selon la revendication 1, dans lequel ledit réceptacle (4406; 4610) comprend un divot pratiquement conique englobant un sommet entièrement positionné, par rapport à la sphère imageable (4402 ; 4602 ; 5008), de sorte qu'un centre d'une image du repère de cliché imageable (4400 ; 4600 ; 5006) coïncide substantiellement avec le sommet du divot.

6. Appareil selon la revendication 1, englobant en outre un capuchon de protection (5014), dimensionné et formé de sorte à protéger le repère de cliché.

7. Appareil selon la revendication 6, dans lequel le capuchon de protection (5014) s'engage dans la tête.

8. Appareil selon la revendication 6, dans lequel le capuchon de protection (5014) englobe une jupe (5020) configurée de sorte à adhérer au cuir chevelu.

9. Appareil selon la revendication 6, dans lequel le capuchon de protection (5024) est ajusté autour de l'arbre.

10. Appareil selon la revendication 1, dans lequel ledit réceptacle (4406 ; 4610) comprend :
une partie proximale filetée (4608) ; et
une partie distale conique.

11. Appareil selon la revendication 1, dans lequel ladite partie de repère de cliché imageable amovible (4404 ; 4604) comprend un cône.

12. Système, comprenant:
l'appareil selon la revendication 1 ;
une source de lumière (3938), configurée de sorte à éclairer les localisateurs de positionnement (4322A-C) de l'instrument de positionnement (4304) ; et
un détecteur (3936), destiné à détecter la lumière réfléchie par les localisateurs de positionnement (4322A-C).

13. Procédé, comprenant les étapes ci-dessous :
fixation (4802) de sphères imageables (4402 ; 4602 ; 5008) sur des bases respectives de plusieurs bases (3904 ; 5002) ayant été vissées (4800) dans au moins une surface d'un sujet ;
formation d'une image (4804) d'un volume englobant les sphères imageables (4402 ; 4602 ; 5002) ;
retrait (4806) des parties imageables respectives (4404 ; 4604 ; 5010) des sphères imageables pour donner accès aux centres (4408 ; 4612 ; 5012) des sphères imageables ; et
mise en repérage (4808) d'un sujet par rapport aux images, englobant l'établissement d'un contact avec les centres des sphères imageables par un instrument de positionnement (4304).
